# EUROPEAN PATENT APPLICATION

(11) **EP 4 488 281 A1**
(43) Date of publication of application: **08.01.2025**
(21) Application number: 23763353.2
(22) Date of filing: 24.02.2023
(51) Int. Cl.: C07H 19/073, A61K 9/06, A61K 9/08, A61K 31/7088, A61K 48/00, A61P 21/00, A61P 21/04, A61P 31/12, A61P 35/00, A61P 43/00, C07H 19/10, C07H 19/173, C07H 21/04, C12N 5/10, C12N 15/10, C12N 15/11, C12N 15/113

(54) **5'-MODIFIED NUCLEOSIDE AND NUCLEOTIDE USING SAME**

(30) Priority: 01.03.2022 JP 2022031341; 11.11.2022 JP 2022181304
(71) Applicant: OSAKA UNIVERSITY, Suita-shi Osaka 565-0871 (JP); Japan As Represented By Director General Of National Institute Of Health Sciences, Kawasaki-shi, Kanagawa, 210-0821 (JP)
(72) Inventor: OBIKA, Satoshi, Suita-shi, Osaka 565-0871 (JP); YAMAGUCHI, Takao, Suita-shi, Osaka 565-0871 (JP); SAKAUE, Shiori, Suita-shi, Osaka 565-0871 (JP); INOUE, Takao, Kawasaki-shi, Kanagawa 210-0821 (JP); YOSHIDA, Tokuyuki, Kawasaki-shi, Kanagawa 210-0821 (JP)
(74) Representative: Schlich
(86) International application number: PCT/JP2023/006672
(87) International publication number: WO 2023/167094

(57) **Abstract**

A 5'-modified nucleoside and a nucleotide using the same are disclosed. The nucleoside of the present invention is a compound represented by a formula (I) below or a salt thereof. The nucleoside of the present invention has a suitable double-strand forming ability for a target RNA and an excellent nuclease-resistant ability and can be produced with high efficiency without the need for an operation for separating diastereomers in the pathway for the synthesis thereof. The nucleoside of the present invention is usable as an alternative to phosphorothioate-modified nucleic acids, which have a risk of, for example, accumulation in a specific organ, and also has high industrial productivity.

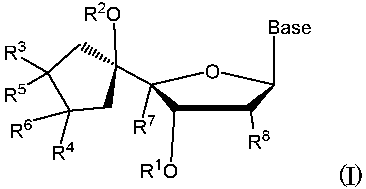

## Description

### Technical Field

The present invention relates to a 5'-modified nucleoside and a nucleotide using the same. More specifically, the invention relates to a 5'-modified nucleoside that has a suitable double-strand forming ability and an excellent nuclease-resistant ability and can be produced with high efficiency, and a nucleotide using the same.

### Background Art

Various artificial nucleic acids have been developed as materials for nucleic acid drugs. In particular, 2',4'-BNA (bridged nucleic acid, also known as LNA) in which the conformation of the sugar moiety in the nucleic acid is fixed through cross-linking has been reported to have excellent binding affinity for a single-stranded RNA (ssRNA) (Non-Patent Documents 1 and 2), and is expected to be suitable for various nucleic acid drugs for antisense therapies and the like.

Meanwhile, an artificial nucleic acid obtained by introducing a methyl group into the 5' position of a nucleic acid has been reported to have excellent properties in terms of the nuclease-resistant ability (Patent Documents 1 to 3). Therefore, an artificial nucleic acid obtained by introducing a substituent into the 5' position is also expected to have applications to diagnosis and medicine. However, synthesis of such an artificial nucleic acid requires separation of diastereomers (Non-Patent Documents 3 and 4), and thus the production process is complicated as a whole.

To address this, in recent years, it has also been proposed to use an artificial nucleic acid obtained without the need for separation of diastereomers by introducing a predetermined cyclopropane structure into the 5' position (such an artificial nucleic acid may be referred to as a "5'-cp-modified nucleic acid" or merely as a "5'-cp" hereinafter) (Patent Document 4). The 5'-cp-modified nucleic acid has an enzyme-resistant ability higher than or equal to that of phosphorothioate-modified nucleic acids (Non-Patent Document 5), which are often used in the current development of nucleic acid drugs, and is expected to be used as an alternative to the phosphorothioate-modified nucleic acids, which have been pointed out to be relevant to hepatotoxicity.

However, the 5'-cp-modified nucleic acid has, for example, an unsatisfactory double-strand forming ability for a target RNA, and is thus desired to be further improved.

### Related Art Documents

### Patent Documents

[Patent Document 1] WO2010/048549
[Patent Document 2] WO2010/048585
[Patent Document 3] WO2010/077578
[Patent Document 4] WO2020/158910

### Non-Patent Documents

[Non-Patent Document 1] S. Obika et al., T. Tetrahedron Lett. 1997, 38, 8735-8738.
[Non-Patent Document 2] S. Singh et al., J. Chem. Commun. 1998, 455-456.
[Non-Patent Document 3] A. M. Yawman et al., J. Org. Chem. 1995, 60, 788-789.
[Non-Patent Document 4] M. Manoharan et al., J. Org. Chem. 2016, 81, 2261-2279.
[Non-Patent Document 5] S. T. Crooke et al., J. Am. Chem. Soc. 2020, 142, 14754-15771.

### Summary of Invention

### Problem to be Solved by the Invention

The present invention was made to address the aforementioned problems, and it is an object thereof to provide a 5'-modified nucleoside that has a suitable double-strand forming ability for a target RNA and an excellent nuclease-resistant ability and can be produced with high efficiency without the need for an operation for separating diastereomers in the pathway for the synthesis thereof, and a nucleotide using the same.

### Means for Solving the Problem

The present invention is a compound represented by a formula (I) below or a salt thereof 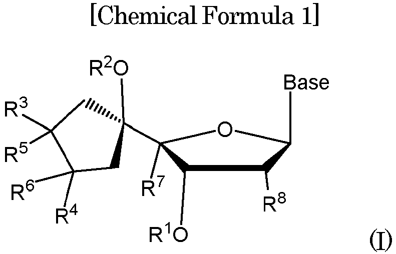 (in the formula (I),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In one embodiment, the formula (I) is represented by a formula (Ia) below: 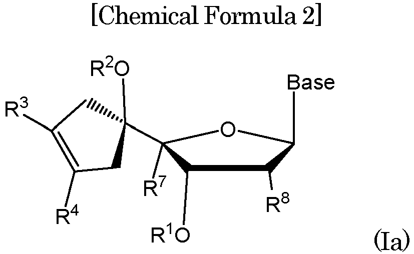 (in the formula (Ia),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In one embodiment, the formula (I) is represented by a formula (Ib) below: 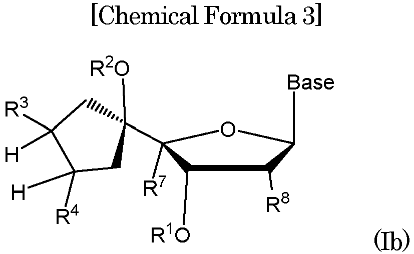 (in the formula (Ib),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In one embodiment, the Base in the formula (I) is a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-ffuoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, or a 4-amino-5-methyl-2-oxo-1,2-dihydropyrimidin-1-yl group.

In one embodiment, the Base in the formula (I) is a group represented by a formula below:

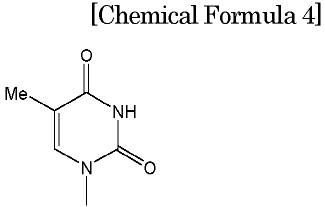

The present invention is also an oligonucleotide containing at least one nucleoside structure represented by a formula (II) below or a pharmacologically acceptable salt thereof 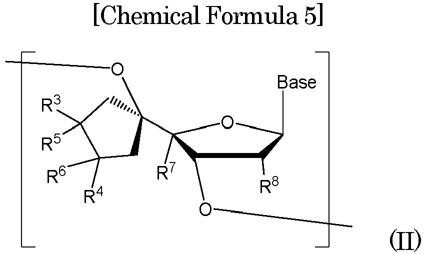 (in the formula (II),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In one embodiment, the formula (II) is represented by a formula (IIa) below: 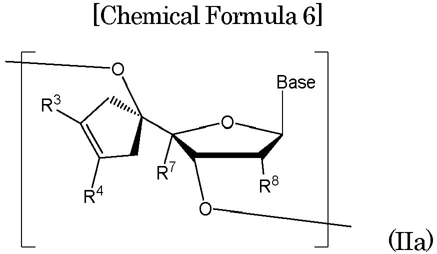 (in the formula (IIa),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In one embodiment, the formula (II) is represented by a formula (IIb) below: 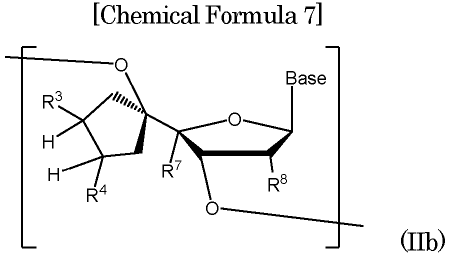 (in the formula (IIb),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

The present invention is also a method for producing the oligonucleotide or pharmacologically acceptable salt thereof according to claim 6, comprising a step of producing an oligonucleotide using a compound represented by a formula (I) below or a salt thereof 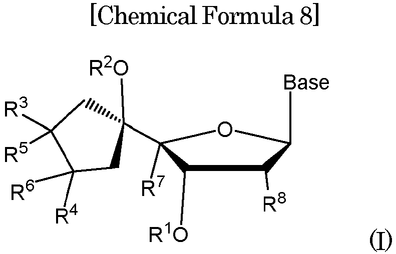 (in the formula (I),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

### Effects of the Invention

With the present invention, provided are a 5'-modified nucleoside having an excellent double-strand forming ability and an excellent enzyme-resistant ability and a nucleotide using the same that are usable in the development of pharmaceuticals. The 5'-modified nucleoside of the present invention is also usable as an alternative to phosphorothioate-modified nucleic acids, which have a risk of, for example, accumulation in a specific organ. The 5'-modified nucleoside of the present invention also has high industrial productivity because diastereomer separation is not required in the production thereof.

### Brief Description of Drawings

FIG. 1 shows evaluation of resistance of five types of oligonucleotides (PO (native), PS, cp, cp2nd, and cp3rd) against an enzyme (3'-exonuclease). The diagram on the left upper side schematically shows 3'-exonuclease and a sequence used in this test, the graph on the left lower side shows the evaluation results, and the diagram on the right side shows the structures of (artificial) nucleic acids contained at the portions T of the oligonucleotides used in the test.
FIG. 2 shows graphs showing the results from analysis of the target gene (GR (NR3C1)) suppressing effect of test oligonucleotides. The results are expressed as the mean ± standard error (S.E.).
FIG. 3 is a graph showing the results from evaluation of AST (U/L) and ALT (U/L) in the blood from test oligonucleotide administration groups. The results are expressed as the mean ± standard error (S.E.).

### Description of Embodiments

The following definitions shall apply throughout the specification.

The term "linear alkyl group having 1 to 6 carbon atoms" as used herein refers to any linear alkyl group having 1 to 6 carbon atoms, and specifically to a methyl group, an ethyl group, an n-propyl group, an n-butyl group, an n-pentyl group, or an n-hexyl group. Meanwhile, the term "alkyl group having 1 to 6 carbon atoms" refers to any linear, branched, or cyclic alkyl group having 1 to 6 carbon atoms.

The term "linear alkoxy group having 1 to 6 carbon atoms" as used herein encompasses alkoxy groups with any linear alkyl group having 1 to 6 carbon atoms. Examples thereof include a methoxy group, an ethoxy group, and an n-propoxy group. Meanwhile, the term "alkoxy group having 1 to 6 carbon atoms" refers to any linear, branched, or cyclic alkoxy group having 1 to 6 carbon atoms. The term "linear alkoxy group having 1 to 6 carbon atoms that may have undergone substitution by a linear alkoxy group having 1 to 6 carbon atoms" refers to the above-mentioned "linear alkoxy group having 1 to 6 carbon atoms" as well as an alkoxy group obtained by substituting one or more hydrogen atoms included in the "linear alkoxy group having 1 to 6 carbon atoms" with another or other "linear alkoxy groups having 1 to 6 carbon atoms" that may be the same or different. Examples of such a "linear alkoxy group having 1 to 6 carbon atoms that may have undergone substitution by a linear alkoxy group having 1 to 6 carbon atoms" include a methoxy group, an ethoxy group, an n-propoxy group, a methoxymethoxy group, an ethoxymethoxy group, an n-propoxymethoxy group, a methoxyethoxy group (e.g., a 2-methoxyethoxy group), an ethoxyethoxy group (e.g., a 2-ethoxyethoxy group), and an n-propoxyethoxy group.

The term "cyanoalkoxy group having 1 to 6 carbon atoms" as used herein refers to a group obtained by substituting at least one hydrogen atom included in any linear, branched, or cyclic alkoxy group having 1 to 6 carbon atoms with a cyano group.

The term "linear alkylthio group having 1 to 6 carbon atoms" as used herein encompasses alkylthio groups with any linear alkyl group having 1 to 6 carbon atoms. Examples thereof include a methythio group, an ethylthio group, and an n-propylthio group. Meanwhile, the term "alkylthio group having 1 to 6 carbon atoms" refers to any linear, branched, or cyclic alkylthio group having 1 to 6 carbon atoms.

The term "linear alkylamino group having 1 to 6 carbon atoms" as used herein encompasses alkylamino groups having one or two alkylamino groups with any linear alkyl group having 1 to 6 carbon atoms. Examples thereof include a methylamino group, a dimethylamino group, an ethylamino group, a methylethylamino group, and a diethylamino group.

The term "alkyl group having 1 to 7 carbon atoms that may be branched or cyclic" as used herein encompasses any linear alkyl groups having 1 to 7 carbon atoms, any branched alkyl groups having 3 to 7 carbon atoms, and any cyclic alkyl groups having 3 to 7 carbon atoms. Such groups may also be referred to merely as "lower alkyl groups". Examples of any linear alkyl groups having 1 to 7 carbon atoms include a methyl group, an ethyl group, an n-propyl group, an n-butyl group, an n-pentyl group, an n-hexyl group, and an n-heptyl group; examples of any branched alkyl groups having 3 to 7 carbon atoms include an isopropyl group, an isobutyl group, a tert-butyl group, and an isopentyl group; and examples of any cyclic alkyl groups having 3 to 7 carbon atoms include a cyclobutyl group, a cyclopentyl group, and a cyclohexyl group.

The term "alkenyl group having 2 to 7 carbon atoms that may be branched or cyclic" as used herein encompasses any linear alkenyl groups having 2 to 7 carbon atoms, any branched alkenyl groups having 3 to 7 carbon atoms, and any cyclic alkenyl groups having 3 to 7 carbon atoms. Such groups may also be referred to merely as "lower alkenyl groups". Examples of any linear alkenyl groups having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group, and a 1-hexenyl group; examples of any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group, and a 1-methyl-2-butenyl group; and examples of any cyclic alkenyl groups having 3 to 7 carbon atoms include a cyclobutenyl group, a cyclopentenyl group, and a cyclohexenyl group.

The term "aryl group having 3 to 10 carbon atoms that may include a heteroatom" as used herein encompasses any aryl groups having 6 to 10 carbon atoms that are constituted by only a hydrocarbon, and any heteroaryl groups having 3 to 12 carbon atoms obtained by substituting at least one carbon atom included in the ring structure of the above-mentioned aryl groups with a heteroatom (e.g., a nitrogen atom, an oxygen atom, and a sulfur atom, and a combination thereof. Examples of the aryl groups having 6 to 10 carbon atoms include a phenyl group, a naphthyl group, an indenyl group, and an azulenyl group; and examples of any heteroaryl groups having 3 to 12 carbon atoms include a pyridyl group, a pyrrolyl group, a quinolyl group, an indolyl group, an imidazolyl group, a furyl group, and a thienyl group.

Examples of the term "aralkyl group with an aryl moiety having 3 to 12 carbon atoms that may include a heteroatom" as used herein include a benzyl group, a phenethyl group, a naphthylmethyl group, a 3-phenylpropyl group, a 2-phenylpropyl group, a 4-phenylbutyl group, a 2-phenylbutyl group, a pyridylmethyl group, an indolylmethyl group, a furylmethyl group, a thienylmethyl group, a pyrrolylmethyl group, a 2-pyridylethyl group, a 1-pyridylethyl group, and a 3-thienylpropyl group.

Examples of the term "acyl group" as used herein include aliphatic acyl groups and aromatic acyl groups. Specifically, examples of the aliphatic acyl groups include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; carboxylated alkylcarbonyl groups such as a succinoyl group, a glutaroyl group, and an adipoyl group; halogeno lower-alkyl-carbonyl groups such as a chloroacetyl group, a dichloroacetyl group, a trichloroacetyl group, and a trifluoroacetyl group; lower-alkoxy-lower-alkyl-carbonyl groups such as a methoxyacetyl group; and unsaturated alkylcarbonyl groups such as an (E)-2-methyl-2-butenoyl group. Examples of the aromatic acyl groups include arylcarbonyl groups such as a benzoyl group, an α-naphthoyl group, and a β-naphthoyl group; halogeno arylcarbonyl groups such as a 2-bromobenzoyl group and a 4-chlorobenzoyl group; low-alkylated arylcarbonyl groups such as a 2,4,6-trimethylbenzoyl group and a 4-toluoyl group; low-alkoxylated arylcarbonyl groups such as a 4-anisoyl group; carboxylated arylcarbonyl groups such as a 2-carboxybenzoyl group, a 3-carboxybenzoyl group, and a 4-carboxybenzoyl group; nitrated arylcarbonyl groups such as a 4-nitrobenzoyl group and a 2-nitrobenzoyl group; low-alkoxycarbonylated arylcarbonyl groups such as a 2-(methoxycarbonyl)benzoyl group; and arylated arylcarbonyl groups such as a 4-phenylbenzoyl group. A formyl group, an acetyl group, a propionyl group, a butyryl group, an isobutyryl group, a pentanoyl group, a pivaloyl group, and a benzoyl group are favorable.

Examples of the term "silyl group" as used herein include tri-lower-alkyl-silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; and tri-lower-alkyl-silyl groups that have undergone substitution by one or two aryl groups, such as a diphenylmethylsilyl group, a butyldiphenylbutylsilyl group, a diphenylisopropylsilyl group, and a phenyldiisopropylsilyl group. A trimethylsilyl group, a triethylsilyl group, a triisopropylsilyl group, a t-butyldimethylsilyl group, and a t-butyldiphenylsilyl group are favorable, and a trimethylsilyl group is more favorable.

Examples of the term "halogen atom" as used herein include a fluorine atom, a chlorine atom, a bromine atom, and an iodine atom. A fluorine atom or a chlorine atom is favorable.

"Protecting groups" in the terms "amino group protecting group for nucleic acid synthesis", "hydroxy group protecting group for nucleic acid synthesis", "hydroxy group protected by a protecting group for nucleic acid synthesis", "phosphate group protected by a protecting group for nucleic acid synthesis", and "mercapto group protected by a protecting group for nucleic acid synthesis" as used herein are not particularly limited as long as they can stably protect an amino group, a hydroxy group, a phosphate group, or a mercapto group during nucleic acid synthesis. Specifically, the protecting groups are stable under an acidic or neutral condition and can be cleaved using chemical techniques such as hydrogenolysis, hydrolysis, electrolysis, and photolysis. Examples of such protecting groups include lower alkyl groups, lower alkenyl groups, acyl groups, tetrahydropyranyl or tetrahydrothiopyranyl groups, tetrahydrofuranyl or tetrahydrothiofuranyl groups, silyl groups, lower-alkoxy-methyl groups, low-alkoxylated lower-alkoxy-methyl groups, halogeno lower-alkoxy-methyl groups, low-alkoxylated ethyl groups, halogenated ethyl groups, methyl groups that have undergone substitution by 1 to 3 aryl groups, "methyl groups that have undergone substitution by 1 to 3 aryl groups in which an aryl ring has undergone substitution by a lower alkyl group, lower alkoxy group, halogen atom, or cyano group", lower-alkoxy-carbonyl groups, "aryl groups that have undergone substitution by a halogen atom, lower alkoxy group, or nitro group", "lower-alkoxy-carbonyl groups that have undergone substitution by a halogen atom or tri-lower-alkyl-silyl group", alkenyloxycarbonyl groups, and "aralkyloxycarbonyl groups in which an aryl ring may have undergone substitution by a lower alkoxy group or nitro group".

More specific examples of the tetrahydropyranyl groups or tetrahydrothiopyranyl groups include a tetrahydropyran-2-yl group, a 3-bromotetrahydropyran-2-yl group, a 4-methoxytetrahydropyran-4-yl group, a tetrahydrothiopyran-4-yl group, and a 4-methoxytetrahydrothiopyran-4-yl group. Examples of the tetrahydrofuranyl groups or tetrahydrothiofuranyl groups include a tetrahydrofuran-2-yl group and a tetrahydrothiofuran-2-yl group. Examples of the lower-alkoxy-methyl groups include a methoxymethyl group, a 1,1-dimethyl-1-methoxymethyl group, an ethoxymethyl group, a propoxymethyl group, an isopropoxymethyl group, a butoxymethyl group, and a t-butoxymethyl group. An example of the low-alkoxylated lower-alkoxy-methyl groups is a 2-methoxyethoxymethyl group. Examples of the halogeno lower-alkoxy-methyl groups include a 2,2,2-trichloroethoxymethyl group and a bis(2-chloroethoxy)methyl group. Examples of the low-alkoxylated ethyl groups include a 1-ethoxyethyl group and a 1-(isopropoxy)ethyl group. An example of the halogenated ethyl groups is a 2,2,2-trichloroethyl group. Examples of the methyl groups that have undergone substitution by 1 to 3 aryl groups include a benzyl group, an α-naphthylmethyl group, a β-naphthylmethyl group, a diphenylmethyl group, a triphenylmethyl group, an α-naphthyldiphenylmethyl group, and a 9-anthrylmethyl group. Examples of the "methyl groups that have undergone substitution by 1 to 3 aryl groups in which an aryl ring has undergone substitution by a lower alkyl group, lower alkoxy group, halogen atom, or cyano group" include a 4-methylbenzyl group, a 2,4,6-trimethylbenzyl group, a 3,4,5-trimethylbenzyl group, a 4-methoxybenzyl group, a 4-methoxyphenyldiphenylmethyl group, a 4,4'-dimethoxytriphenylmethyl group, a 2-nitrobenzyl group, a 4-nitrobenzyl group, a 4-chlorobenzyl group, a 4-bromobenzyl group, and a 4-cyanobenzyl group. Examples of the lower-alkoxy-carbonyl groups include a methoxycarbonyl group, an ethoxycarbonyl group, a t-butoxycarbonyl group, and an isobutoxycarbonyl group. Examples of the "aryl groups that have undergone substitution by a halogen atom, lower alkoxy group, or nitro group" include a 4-chlorophenyl group, a 2-fluorophenyl group, a 4-methoxyphenyl group, a 4-nitrophenyl group, and a 2,4-dinitrophenyl group. Examples of the "lower-alkoxy-carbonyl groups that have undergone substitution by a halogen atom or tri-lower-alkyl-silyl group" include a 2,2,2-trichloroethoxycarbonyl group and 2-trimethylsilylethoxycarbonyl group. Examples of the alkenyloxycarbonyl groups include a vinyloxycarbonyl group and an aryloxycarbonyl group. Examples of the "aralkyloxycarbonyl groups in which an aryl ring may have undergone substitution by a lower alkoxy group or nitro group" include a benzyloxycarbonyl group, a 4-methoxybenzyloxycarbonyl group, a 3,4-dimethoxybenzyloxycarbonyl group, a 2-nitrobenzyloxycarbonyl group, and a 4-nitrobenzyloxycarbonyl group.

In an embodiment, examples of the "hydroxy group protecting group for nucleic acid synthesis" include aliphatic acyl groups, aromatic acyl groups, methyl groups that have undergone substitution by 1 to 3 aryl groups, "methyl groups that have undergone substitution by 1 to 3 aryl groups in which an aryl ring has undergone substitution by a lower alkyl, lower alkoxy, halogen, or cyano group", and silyl groups. Alternatively, in an embodiment, examples of the "hydroxy group protecting group for nucleic acid synthesis" include an acetyl group, a benzoyl group, a benzyl group, a p-methoxybenzoyl group, a dimethoxytrityl group, a monomethoxytrityl group, a tert-butyldiphenylsilyl group, a tert-butyldimethylsilyl (TBDMS) group, a [(triisopropylsilyl)oxy]methyl (TOM) group, a [(2-nitrobenzyl)oxy]methyl (NBOM) group, a bis(acetoxyethoxy)methyl ether (ACE) group, a tetrahydro-4-methoxy-2H-pyran-2-yl (Mthp) group, a 1-(2-cyanoethoxy)ethyl (CEE) group, a 2-cyanoethoxymethyl (CEM) group, a tert-butyldithiomethyl (DTM) group, a 2-(4-tolylsulfonyl)ethoxymethyl (TEM) group, and a 4-(N-dichloroacetyl-N-methylamino)benzyloxymethyl (4-MABOM) group.

In an embodiment, examples of the protecting group used for the "hydroxy group protected by a protecting group for nucleic acid synthesis" include aliphatic acyl groups, aromatic acyl groups, "methyl groups that have undergone substitution by 1 to 3 aryl groups", "aryl groups that have undergone substitution by a halogen atom, lower alkoxy group, or nitro group", lower alkyl groups, and lower alkenyl groups. Alternatively, in an embodiment, examples of the protecting group used for the "hydroxy group protected by a protecting group for nucleic acid synthesis" include a benzoyl group, a benzyl group, a 2-chlorophenyl group, a 4-chlorophenyl group, and a 2-propenyl group.

In an embodiment, examples of the "amino group protecting group for nucleic acid synthesis" include acyl groups, and a benzoyl group is favorable.

In an embodiment, examples of the "protecting group" used for the "phosphate group protected by a protecting group for nucleic acid synthesis" include lower alkyl groups, lower alkyl groups that have undergone substitution by a cyano group, aralkyl groups, "aralkyl groups in which an aryl ring has undergone substitution by a nitro group or halogen atom", and "aryl groups that have undergone substitution by a lower alkyl group, halogen atom, or nitro group". Alternatively, in an embodiment, examples of the "protecting group" used for the "phosphate group protected by a protecting group for nucleic acid synthesis" include a 2-cyanoethyl group, a 2,2,2-trichloroethyl group, a benzyl group, a 2-chlorophenyl group, and a 4-chlorophenyl group.

In an embodiment, examples of the "protecting group" used for the "mercapto group protected by a protecting group for nucleic acid synthesis" include aliphatic acyl groups and aromatic acyl groups, and a benzoyl group is favorable.

In this specification, among groups represented by -P(R⁴)R⁵ [where R⁴ and R⁵ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has an alkyl group having 1 to 6 carbon atoms], a group in which R⁴ is OR^{4a} and R⁵ is NR^{5a} is referred to as a "phosphoramidite group" (where an example of R^{4a} is a cyanoalkoxy group having 1 to 6 carbon atoms, and an example of R^{5a} is an alkyl group having 1 to 6 carbon atoms). Favorable examples of the phosphoramidite group include a group represented by a formula -P(OC₂H₄CN)(N(iPr)₂) and a group represented by a formula -P(OCH₃)(N(iPr)₂). In these formulae, iPr represents an isopropyl group.

The terms "nucleoside" and "nucleoside analogue" as used herein refer to non-naturally occurring nucleosides of "nucleosides" in which a purine base or a pyrimidine base binds to sugar, as well as those in which a heteroaromatic ring or an aromatic hydrocarbon ring other than purine bases and pyrimidine bases that can serve as a substitute for a purine base or pyrimidine base binds to sugar.

The terms "artificial oligonucleotide" and "oligonucleotide analogue" as used herein refer to non-naturally occurring derivatives of "oligonucleotides" in which, for example, two to fifty of the same or different "nucleosides" or "nucleoside analogues" are bound via phosphodiester bonds. Favorable examples of such analogues include sugar derivatives with sugar moieties modified; thioated derivatives with phosphate diester moieties thioated; esters with terminal phosphate moieties esterified; and amides in which amino groups on purine bases are amidated, and the sugar derivatives with sugar moieties modified are more favorable.

The term "salt thereof" as used herein refers to a salt of a compound represented by the formula (I) of the present invention. Examples of such a salt include metal salts including alkali metal salts such as sodium salts, potassium salts, and lithium salts, alkali earth metal salts such as calcium salts and magnesium salts, aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including halide hydroacid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

The term "pharmacologically acceptable salt thereof" refers to a salt of an oligonucleotide analogue containing at least one nucleoside structure represented by the formula (II) of the present invention. Examples of such a salt include metal salts including alkali metal salts such as sodium salts, potassium salts, and lithium salts, alkali earth metal salts such as calcium salts and magnesium salts, aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including halide hydroacid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

Hereinafter, the present invention will be described in detail.

A 5'-modified nucleoside of the present invention is a compound represented by the formula (I) below or a salt thereof 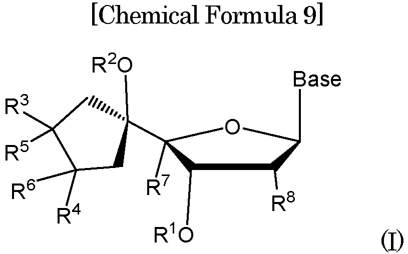 (in the formula (I),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

In the formula (I) above, "Base" is, for example, a purine base (i.e., a purin-9-yl group) or a pyrimidine base (i.e., a 2-oxo-1,2-dihydropyrimidin-1-yl group). These bases may have any one or more substituents selected from the α group consisting of a hydroxy group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms, and halogen atoms.

Specific examples of the "Base" above include an adeninyl group, a guaninyl group, a cytosinyl group, an uracinyl group, a thyminyl group, a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-ffuoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, and a 4-amino-5-methyl-2-oxo-1,2-dihydropyrimidin-1-yl group.

Alternatively, from the viewpoint of introduction into a nucleic acid drug, the "Base" is preferably a group represented by a structural formula below: 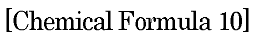 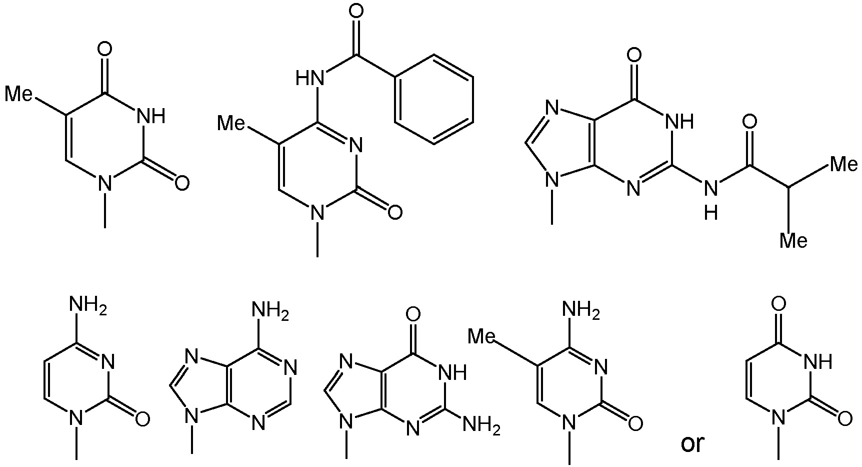 or a 2-oxo-4-hydroxy-5-methyl-1,2-dihydropyrimidin-1-yl group, a 2-oxo-4-amino-1,2-dihydropyrimidin-1-yl group, a 6-aminopurin-9-yl group, a 2-amino-6-hydroxypurin-9-yl group, a 4-amino-5-methyl-2-oxo-1,2-dihydropyrimidin-1-yl group, or a 2-oxo-4-hydroxy-1,2-dihydropyrimidin-1-yl group. It is preferable that a hydroxy group and an amino group included in the above-mentioned groups serving as the "Base" are protected by a protecting group during oligonucleotide synthesis.

The 5'-modified nucleoside of the present invention has a five-membered ring structure such as a cyclopentene structure or cyclopentane structure at the 5' position of the formula (I). With this structure, the nucleoside of the present invention cannot have a diastereomer structure at the 5' position, and therefore, compared with conventional artificial nucleic acids obtained by introducing a substituent into the 5' position, the separation of diastereomers during synthesis is no longer necessary.

In an embodiment, from the viewpoint of combinations of R⁵ and R⁶ in the formula (I) above, examples of the formula (I) above include: 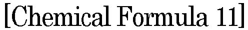 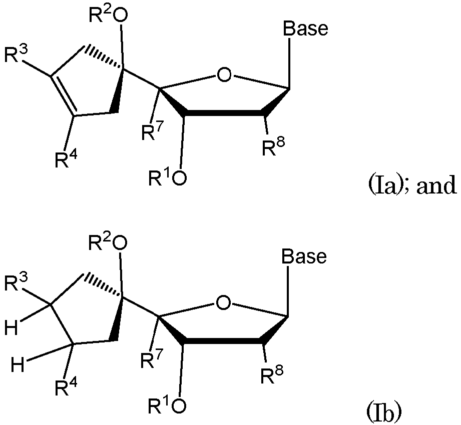 (in the formulae (Ia) and (Ib), the Base, R¹, R², R³, R⁴, R⁷, and R⁸ are as defined for the formula (I) above). More specific examples of the formula (I) above include: 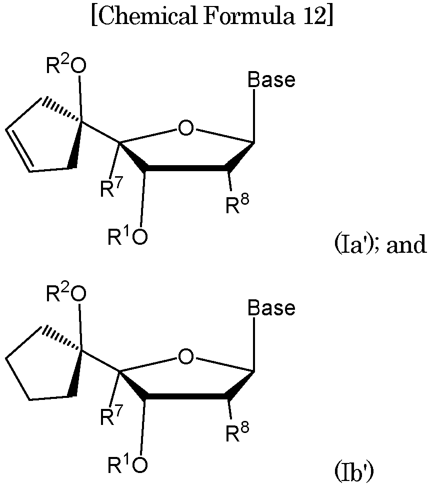 (in the formulae (Ia') and (Ib'), the Base, R¹, R², R⁷, and R⁸ are as defined for the formula (I) above). In this specification, a compound represented by the formula (Ia') above may be referred to as "a 5'-cp2 (modified nucleic acid)" and a compound represented by the formula (Ib') may be referred to as "a 5'-cp3 (modified nucleic acid)".

Alternatively, in an embodiment, from the viewpoint of combinations of R⁷ and R⁸ in the formula (I) above, examples of the formula (I) above include: 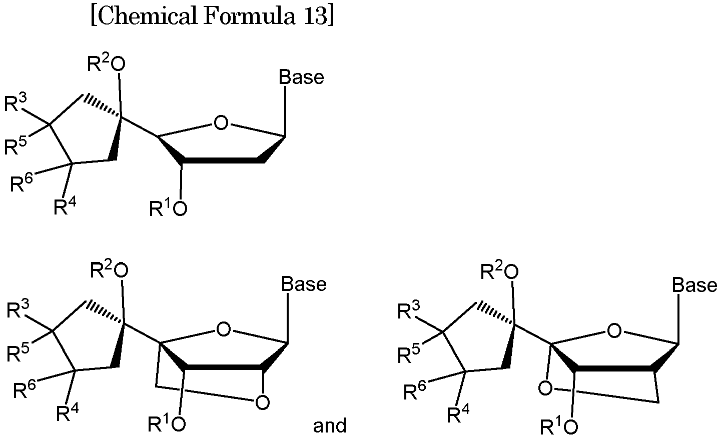 (where the Base, R¹, R², R³, R⁴, R⁵, and R⁶ are as defined for the formula (I) above).

Alternatively, in an embodiment, from the viewpoint of combinations of R⁵, R⁶, R⁷, and R⁸ in the formula (I) above, examples of the formula (I) above include: 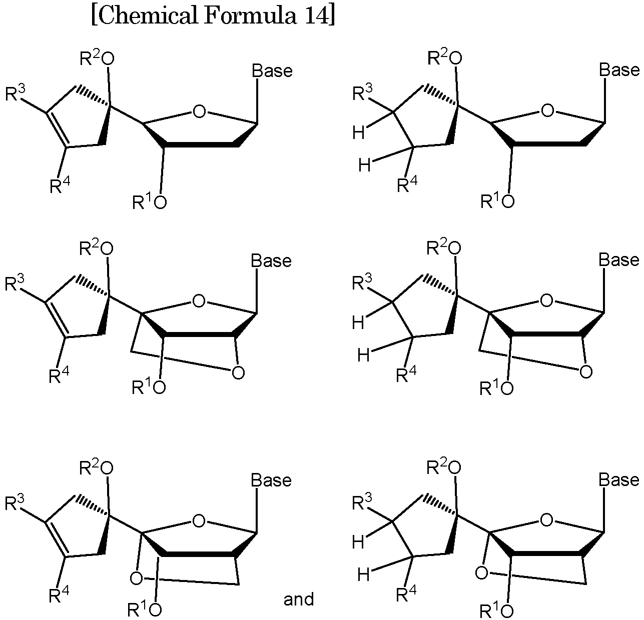 (where the Base, R¹, R², R³, and R⁴ are as defined for the formula (I) above).

The 5'-modified nucleoside of the present invention improves the nuclease-resistant ability of an oligonucleotide, which will be described later, due to a predetermined five-membered ring structure as described above being introduced at the 5' position of the formula (I). Moreover, a distortion of the ring of such a five-membered ring structure is small and has little effect on the phosphate backbone. Accordingly, oligonucleotides containing the 5'-modified nucleoside of the present invention exhibit an excellent double-strand formation ability for a target RNA.

In the present invention, an oligonucleotide can be easily produced by using such a 5'-modified nucleoside of the formula (I), and using, for example, an amidite method that is well known in the art, or triphosphorylation such as that described in M. Kuwahara et al., Nucleic Acids Res., 2008, Vol. 36, No. 13, pp. 4257-4265.

The oligonucleotide of the present invention or a pharmacologically acceptable salt thereof (these may be collectively referred to as an "oligonucleotide of the present invention" hereinafter) contains at least one nucleoside structure represented by a formula (II) below: 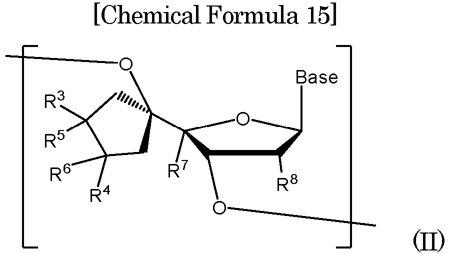 (in the formula (II),
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together.

In an embodiment, from the viewpoint of combinations of R⁶ and R⁷, examples of the nucleoside structure of the formula (II) contained in the oligonucleotide of the present invention include: 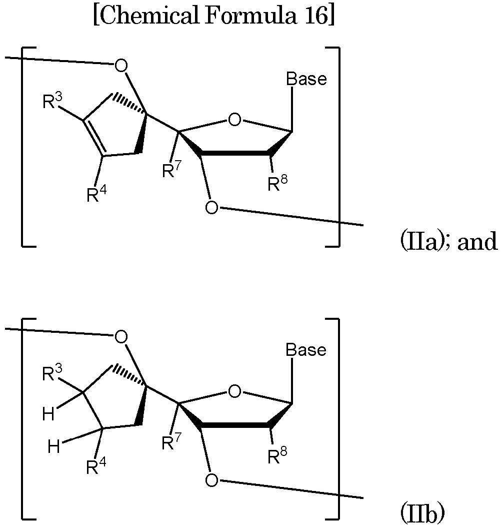 (in the formulae (IIa) and (IIb), the Base, R³, R⁴, R⁷, and R⁸ are as defined for the formula (I) above). More specific examples of the nucleoside structure of the formula (II) above contained in the oligonucleotide of the present invention include: 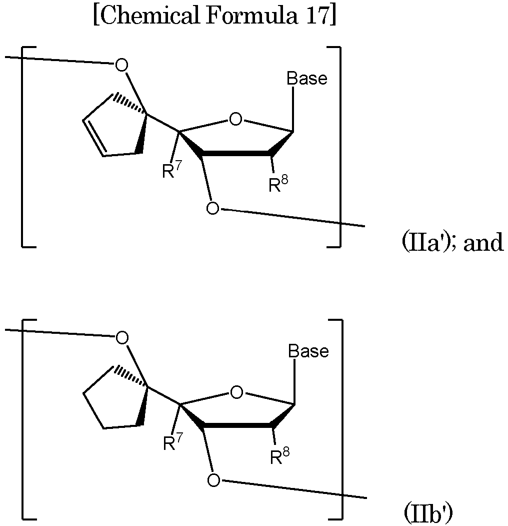 (in the formulae (IIa') and (IIb'), the Base, R⁷, and R⁸ are as defined for the formula (I) above).

Alternatively, in an embodiment, from the viewpoint of combinations of R⁷ and R⁸ in the formula (II) above, examples of the nucleoside structure of the formula (II) contained in the oligonucleotide of the present invention include: 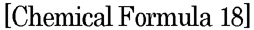 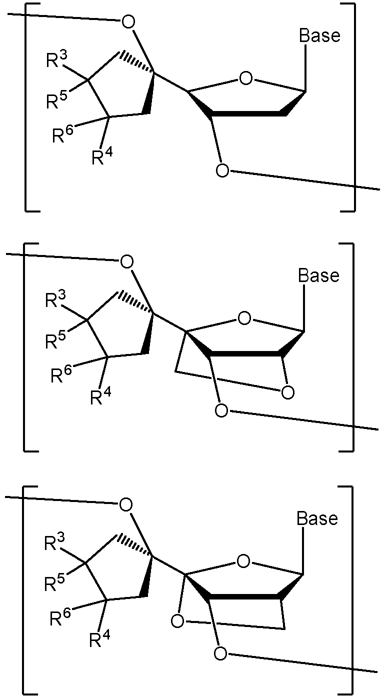 and
(where the Base, R³, R⁴, R⁵, and R⁶ are as defined for the formula (I) above).

Alternatively, in an embodiment, from the viewpoint of combinations of R⁵, R⁶, R⁷, and R⁸ in the formula (II) above, examples of the nucleoside structure of the formula (II) contained in the oligonucleotide of the present invention include: 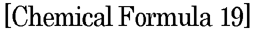 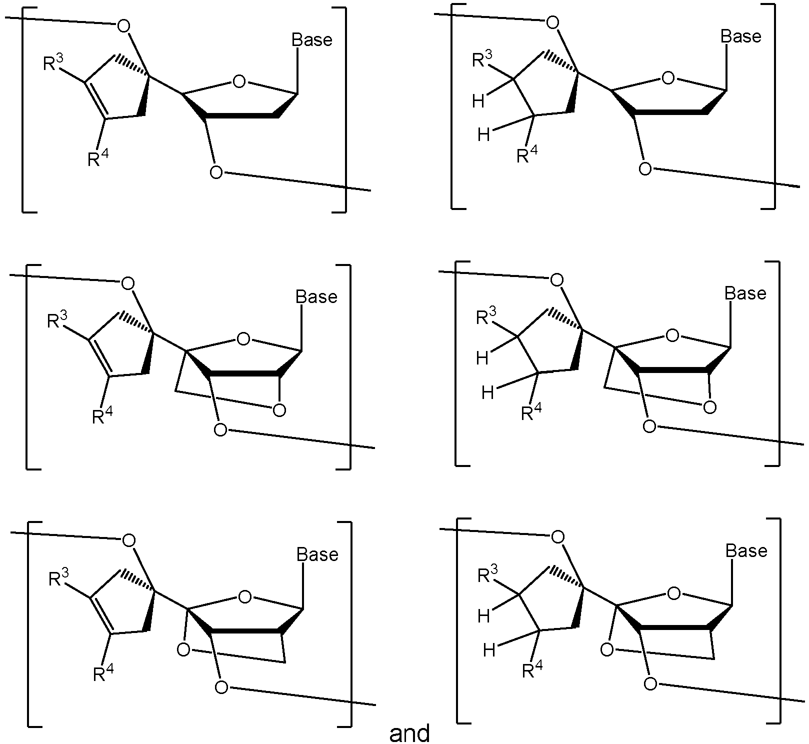 (where the Base, R³, and R⁴ are as defined for the formula (I) above).

The oligonucleotide of the present invention has at least one of the above-described nucleoside structures at any position. There is no particular limitation on the positions and number of the nucleoside structures, and the oligonucleotide can be designed as appropriate depending on the purpose.

An oligonucleotide (antisense molecule) containing such a nucleoside structure has a significantly improved nuclease-resistant ability compared with the conventional cases where 2',4'-BNA/LNA is used. Also, such an oligonucleotide has an appropriate double-strand forming ability for a target RNA. Furthermore, with the oligonucleotide, it is possible to reduce hepatotoxicity without impairing antisense activity.

With all these facts, the oligonucleotide of the present invention synthesized using the 5'-modified nucleoside above is expected to be useful as a pharmaceutical (antisense molecule) inhibiting or restoring the functions of specific genes, such as antitumor agents and antiviral drugs, to treat a disease. An example of such a pharmaceutical that is expected to be useful is a therapeutic drug for muscular dystrophy.

In particular, for antisense therapies, the binding affinity (double-strand forming ability) for a complementary sense strand RNA and the resistance to in-vivo nucleic acid-degrading enzymes (nucleases) are both required. In particular, it is essential to production of a pharmaceutical using a nucleic acid molecule that the nuclease-resistant ability is imparted to the nucleic acid molecule. The 5'-modified nucleic acid of the present invention has a nuclease-resistant ability higher than or equal to that of phosphorothioate-modified nucleic acids, which are often used in the current development of nucleic acid drugs, and is expected to be used as an alternative to the phosphorothioate-modified nucleic acids, which have been pointed out to be relevant to hepatotoxicity.

Additives typically used in the art of pharmaceuticals such as excipients, binders, preservatives, oxidation stabilizers, disintegrants, lubricants, and flavoring substances can be added to the oligonucleotide of the present invention to prepare parenteral formulations or liposomal formulations. Also, for example, topical formulations such as liquids, creams, and ointments may be prepared by adding pharmaceutical carriers typically used in the art.

### Examples

Hereinafter, the present invention will be described in greater detail using examples, but the present invention is not limited to the examples below.

### Example 1: Synthesis of 5'-Modified Nucleoside (1)

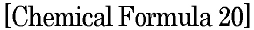

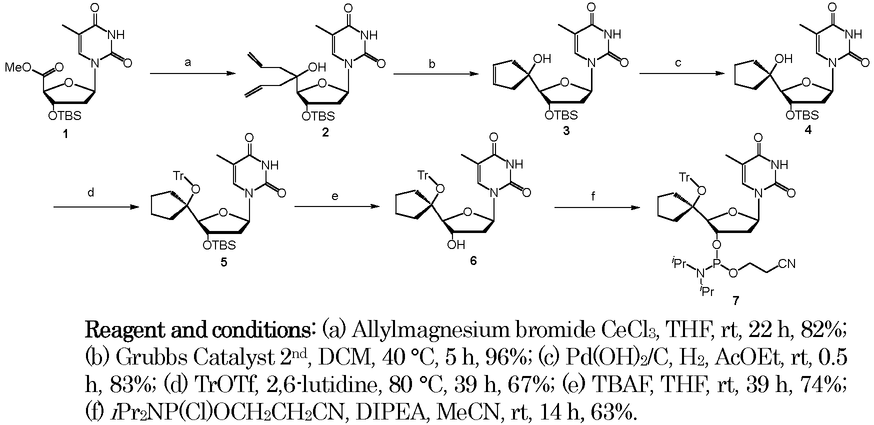

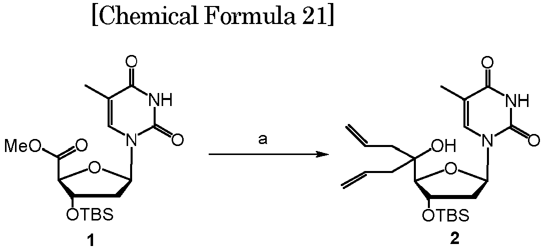

Cerium (III) chloride (1287 mg, 5.22 mmol) was added to a compound 1 (500 mg, 1.30 mmol) produced using the method described in Patent Document 4 above (WO 2020/158910) and toluene azeotropy was conducted. Then, a suspension was produced by adding anhydrous tetrahydrofuran (13 mL), and a 1.0 M diethyl ether solution of allylmagnesium bromide (5.2 mL, 5.2 mmol) was dripped to the suspension at room temperature under a nitrogen stream. After the dripping was completed, the suspension was stirred for another 1 hour. After the reaction was completed, a saturated aqueous solution of ammonium chloride was added to the suspension, the resulting mixture was filtered through cerite, and the filtrate was subjected to extraction with ethyl acetate. The organic layer was washed with water and a saturated saline solution and was then dried over anhydrous sodium sulfate, and the solvent was distilled away under reduced pressure. The obtained residue was purified through silica gel column chromatography (SiO₂, hexane : ethyl acetate = 2 : 1) to obtain a compound 2 (463 mg, 82%) as a yellow solid. Table 1 shows data on the properties of the obtained compound 2.

**[Table 1]**

| Physical property data of the obtained compound 2 |
|---|
| ¹H NMR (300 MHz, CDCl₃) δ 0.11 (s, 6H), 0.90 (s, 9H), 1.93 (d, *J* = 1.0 Hz, 3H), 2.10-2.18 (m, 1H), 2.25-2.49 (m, 5H), 2.56 (bs, 1H), 3.81 (d, *J* = 2.4 Hz, 1H), 4.59-4.63 (m, 1H), 5.11-5.27 (m, 4H), 5.76-5.97 (m, 2H), 6.16 (dd, *J* = 6.2, 8.3 Hz, 1H), 7.52 (d, *J* = 1.4 Hz, 1H), 8.32 (bs, 1H); ¹³C NMR (76 MHz, CDCl₃) δ -4.6, -4.1, 12.7, 17.9, 25.8, 39.9, 40.6, 40.6, 71.9, 74.3, 87.0, 90.9, 111.1, 119.5, 120.0, 132.5, 133.3, 137.6, 150.4, 163.6; HRMS (MALDI): Calculated for C₂₂H₃₆N₂O₅NaSi [M+Na]⁺: 459.2285 Found: 459.2286. |

### (1-2) Synthesis of Compound 3

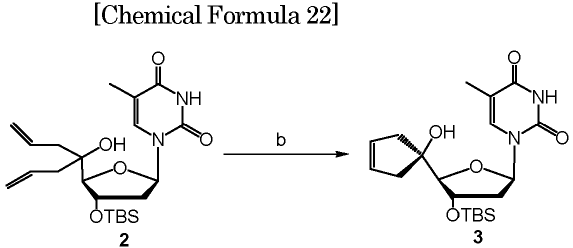

A second-generation Grubbs catalyst (71.6 mg, 0.0840 mmol) was added to a deoxygenated dichloromethane solution (56 mL) of the compound 2 obtained above (736 mg, 1.69 mmol) under a nitrogen stream. After the mixture was stirred at 40°C for 3 hours, dichloromethane was distilled away under reduced pressure, and the obtained crude product was purified through silica gel column chromatography (SiO₂, hexane : ethyl acetate = 3 : 2) to obtain a compound 3 (660 mg, 96%) as a white solid. Table 2 shows data on the properties of the obtained compound 3.

**[Table 2]**

| Physical property data of the obtained compound 3 |
|---|
| ¹H NMR (300 MHz, CDCl₃) δ 0.10 (s, 6H), 0.90 (s, 9H), 1.91 (s, 3H), 2.09-2.17 (m, 1H), 2.33-2.75 (m, 5H), 2.93 (bs, 1H), 3.89 (d, *J* = 2.1 Hz, 1H), 4.46--4.53 (m, 1H), 5.70-5.78 (m, 2H), 6.13 (dd, *J* = 6.2, 8.2 Hz, 1H), 7.50 (s, 1H), 8.62 (bs, 1H); ¹³C NMR (76 MHz, CDCl₃) δ -4.7, -4.3,12.7, 17.9, 25.8, 40.6, 44.0, 46.2,72.6, 81.1, 87.5, 93.5,111.0, 128.1, 129.0,137.8, 150.4, 163.7; HRMS (MALDI): Calculated for C₂₀H₃₂N₂O₅NaSi [M+Na]⁺: 431.1970 Found: 431.1973. |

### (1-3) Synthesis of Compound 4

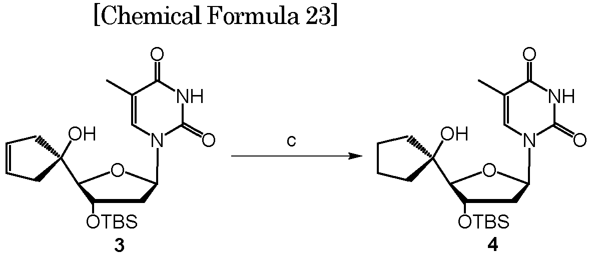

Palladium hydroxide-activated carbon (Pd 20%) (100 mg, 50 wt%) was added to an ethyl acetate solution (4.9 mL) of the compound 3 obtained above (200 mg, 0.488 mmol). The flask was purged with hydrogen and the reaction solution was stirred at room temperature for 0.5 hours. After the reaction was completed, the reaction solution was filtered, and ethyl acetate was distilled away under reduced pressure. The obtained residue was purified through silica gel column chromatography (SiO₂, hexane : ethyl acetate = 3 : 2) to obtain a compound 4 (166 mg, 83%) as a white solid. Table 3 shows data on the properties of the obtained compound 4.

**[Table 3]**

| Physical property data of the obtained compound 4 |
|---|
| ¹H NMR (300 MHz, CDCl₃) δ 0.09 (s, 3H), 0.10 (s, 3H), 0.90 (s, 9H), 1.61-1.90 (m, 8H), 1.92 (d, *J* = 1.0, 3H), 2.09 (ddd, J = 2.4, 5.9,13.1 Hz, 1H), 2.50 (ddd, *J* = 6.5, 8.6, 14.7 Hz, 1H),2.79 (s, 1H), 3.75 (d, *J* = 2.4 Hz, 1H), 4.48-4.53 (m, 1H), 6.02 (dd, *J* = 6.2, 8.6 Hz, 1H), 7.32 (d, *J* = 1.4 Hz, 1H), 8.59 (s, 1H); ¹³C NMR (76 MHz, CDCl₃) δ -4.7, -4.3,12.6,17.9,23.9,24.2,25.8, 36.4, 39.2, 40.3, 72.6, 82.3, 88.2, 94.0, 111.2, 138.1, 150.4, 163.7; HRMS (MALDI): Calculated for C₂₀H₃₄N₂O₅NaSi [M+Na]⁺: 433.2129 Found: 433.2129. |

### (1-4) Synthesis of Compound 5

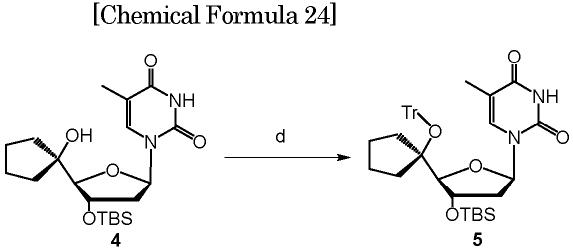

Silver trifluoromethanesulfonate (2060 mg, 8.02 mmol) was added to an anhydrous dichloromethane solution (13 mL) of trityl chloride (2242 mg, 8.04 mmol) and mixed. After turning orange, the suspension was stirred at room temperature for 3 hours, and then silver chloride was precipitated. The supernatant, a trityl triflate solution, (6 mL) was added to an anhydrous 2,6-lutidine solution (6 mL) of the compound 4 obtained above (488 mg, 1.19 mmol) at 0°C. After the mixture was stirred at 80°C for 39 hours, a saturated aqueous solution of sodium hydrogen carbonate was added to the reaction solution. After a saturated aqueous solution of copper sulfate was further added to emulsify lutidine, the resulting mixture was filtered through cerite using ethyl acetate. The solvent was distilled away under reduced pressure, and then the obtained residue was purified through silica gel column chromatography (SiO₂, containing 0.5% triethylamine, hexane : ethyl acetate = 7 : 3) to obtain a compound 5 (523 mg, 67%) as a yellow solid. Table 4 shows data on the properties of the obtained compound 5.

**[Table 4]**

| Physical property data of the obtained compound 5 |
|---|
| ¹H NMR (300 MHz, CDCl₃) δ 0.05 (s, 3H), 0.12 (s, 3H), 0.88 (s, 9H), 1.08-1.34 (m, 4H), 1.37 (s, 3H), 1.40-1.53 (m, 2H), 1.79-190 (m, 2H), 2.23-2.34 (m, 2H), 3.51 (d, *J* = 3.8 Hz, 1H), 4.86-4.92 (m, 1H), 6.35 (dd, *J* = 6.9 Hz, 1H), 7.21-7.31 (m, 9H), 7.43 (d, *J* = 1.0 Hz, 1H), 7.57-7.63 (m, 6H), 8.32 (s, 1H); ¹³C NMR (76 MHz, CDCl₃) δ -4.8, -3.9, 12.0, 17.8, 23.6, 24.7, 25.8, 33.7, 41.6, 71.2, 82.8, 85.8, 89.7, 91.7, 111.4, 127.2, 127.6, 129.4, 136.1, 146.2, 150.5, 163.6; HRMS (MALDI): Calculated for C₃₉H₄₈N₂O₅NaSi [M+Na]⁺: 675.3254 Found: 675.3224. |

### (1-5) Synthesis of Compound 6

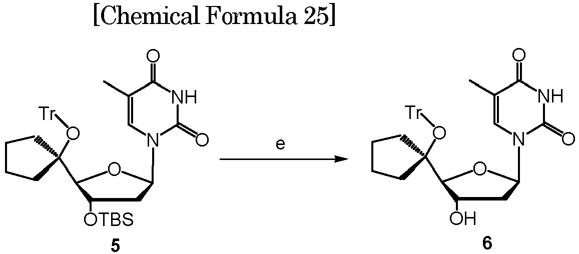

A 1.0 M tetrahydrofuran solution of tetrabutylammonium fluoride (345 pL, 0.345 mmol) was added to a tetrahydrofuran solution (3.8 mL) of the compound 5 obtained above (225 mg, 0.345 mmol), and the resulting mixture was stirred at room temperature for 39 hours. After the reaction was completed, the solvent was distilled away under reduced pressure, and then the obtained residue was purified through silica gel column chromatography (SiO₂, containing 0.5% triethylamine, chloroform : methanol = 30 : 1 → hexane : ethyl acetate = 1 : 1) to obtain a compound 6 (161 mg, 87%) as a white solid. Table 5 shows data on the properties of the obtained compound 6.

**[Table 5]**

| Physical property data of the obtained compound 6 |
|---|
| ¹H NMR (500 MHz, CDCl₃) δ 1.02-1.14 (m, 1H), 1.20-1.51 (m, 5H), 1.56 (d, *J* = 1.2 Hz, 3H), 1.84 -1.90 (m, 1H), 1.96 (d, *J* = 1.7 Hz, 1H), 1.98-2.06 (m, 1H), 2.22-2.33 (m, 2H), 3.42 (d, *J* = 4.4 Hz, 1H), 4.81-1.85 (m, 1H), 6.24 (dd, *J* = 6.1, 8.0 Hz, 1H), 7.21-7.30 (m, 10H), 7.54-7.57 (m, 6H), 8.13 (s, 1H); ¹³C NMR (76 MHz, CDCl₃) δ 12.3, 25.0, 26.8, 34.5, 35.4, 40.7, 71.3, 83.0, 86.4, 90.0, 90.9, 111.6, 127.3, 127.8, 129.3, 135.7, 146.0, 150.5, 163.6; HRMS (MALDI): Calculated for C₃₃H₃₄N₂O₅NaSi [M+Na]⁺: 561.2360 Found: 561.2356. |

### (1-6) Synthesis of Compound 7 (Amidite Block 7)

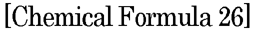

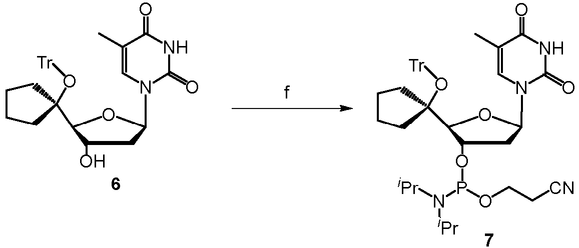

N,N-diisopropylethylamine (400 µL, 2.30 mmol) and 2-cyanoethyl-N,N-diisopropyl phosphorochloridate (245 pL, 1.10 mmol) were successively added to an anhydrous acetonitrile solution (7.4 mL) of the compound 6 obtained above (397 mg, 0.737 mmol) at 0°C under a nitrogen stream, and the resulting mixture was stirred at room temperature for 2.5 hours. After the reaction was completed, the solvent was distilled away under reduced pressure, and then the obtained residue was purified through silica gel column chromatography (SiO₂, containing 0.5% triethylamine, hexane : ethyl acetate = 3 : 2). Furthermore, reprecipitation (toluene/hexane) was conducted to obtain a compound 7 (344 mg, 63%, diastereomer ratio 3 : 2) as a white solid. Table 6 shows data on the properties of the obtained compound 7.

**[Table 6]**

| Physical property data of the obtained compound 7 |
|---|
| ¹H NMR (300 MHz, CDCl₃) δ 0.78-0.97 (m, 1H), 1.09-1.56 (m, 21H), 1.75-1.97 (m, 2H), 2.27-2.71 (m, 4H), 3.51-3.96 (m, 5H) 4.98-5.10 (m, 1H), 6.40 (dd, *J* = 5.5, 8.9 Hz, 1H), 7.17-7.30 (m, 8H), 7.30-7.37 (m, 2H), 7.52-7.66 (m, 6H), 8.41 (bs, 1H); ³¹P NMR (121.7 MHz, CDCl₃) δ 147.9, 148.7; HRMS (MALDI): Calculated for C₄₂H₅₁N₄O₆NaSi [M+Na]⁺: 761.3437 Founds: 761.3438. |

### Example 2: Synthesis of 5'-Modified Nucleoside (2)

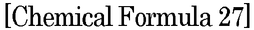

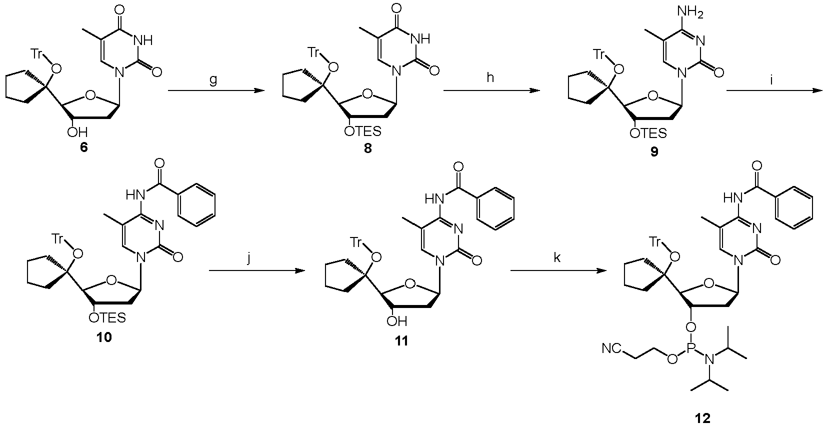

Reagent and conditions: (g) TESCI, pyridine, rt, 1 h, 82%; (h) 1,2,4-triazole, POCl₃, DCM, rt, 2.5 h; 28% aq. NH₃, 1,4-dioxane, rt, 2.5 h, 93%; (i) Bz₂O, pyridine, rt, 16 h, 97%; (j) TBAF, THF, rt, 1 h, 67%; (k) iPr₂NP(Cl)OCH₂CH₂CN, DIPEA, DCM, rt, 5.5 h, 83%.

### (2-1) Synthesis of Compound 8

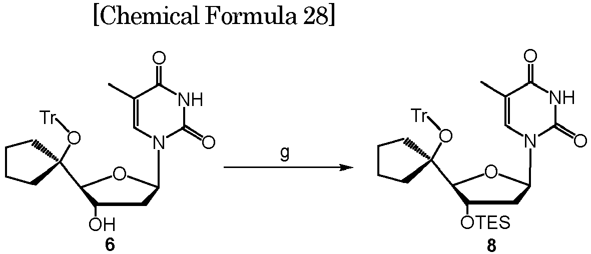

Chlorotriethylsilane (320 pL, 2.48 mmol) was added to a pyridine solution (6.2 mL) of the compound 6 obtained above (333 mg, 0.618 mmol) under a nitrogen stream, and the resulting mixture was stirred at room temperature for 1 hour. After the reaction was completed, a saturated aqueous solution of sodium hydrogen carbonate was added, the resulting mixture was subjected to extraction with ethyl acetate, washed with water and a saturated saline solution, and dried over anhydrous sodium sulfate, and then the solvent was distilled away under reduced pressure. The obtained residue was purified through silica gel column chromatography (SiO₂, containing 0.5% triethylamine, hexane : ethyl acetate = 3 : 1) to obtain a compound 8 (332 mg, 82%) as a white solid. Table 7 shows data on the properties of the obtained compound 8.

**[Table 7]**

| Physical property data of the obtained compound 8 |
|---|
| ¹H NMR (300 MHz, CDCl₃) δ 0.56-0.65 (m, 6H), 0.74-0.84 (m, 1H), 0.92-0.99 (m, 9H), 1.08-1.30 (m, 3H), 1.33 (s, 3H), 1.36-1.43 (m, 1H), 1.45-1.52 (m, 1H), 1.81-1.88 (m, 2H), 2.24-2.35 (m, 2H), 3.57 (d, *J* = 1.5 Hz, 1H), 4.94-4.98 (m, 1H), 6.40 (dd, *J* = 6.0, 6.0 Hz, 1H), 7.23-7.26 (m, 9H), 7.40 (s, 1H), 7.59 (d, *J* = 8.0 Hz, 6H), 8.56 (bs, 1H); ¹³C NMR (126 MHz, CDCl₃) δ 5.0, 6.4, 12.0, 23.5, 24.6, 33.4, 33.8, 41.7, 71.2, 82.8, 85.6, 89.7, 91.9, 111.4, 127.2, 127.6, 129.4, 136.1, 146.1, 150.1, 163.9; HRMS (MALDI): Calculated for C₃₉H₄₈N₂O₅NaSi [M+Na]⁺: 675.3247 Found: 675.3225. |

### (2-2) Synthesis of Compound 9

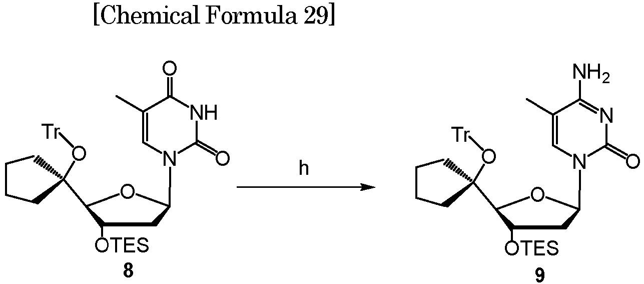

Triethylamine (160 pL, 1.15 mmol), 1,2,4-triazole (79 mg, 1.15 mmol), and phosphoryl chloride (21 pL, 0.230 mmol) were successively added to an anhydrous dichloromethane solution (760 pL) of the compound 8 obtained above (50 mg, 0.0766 mmol) at 0°C under a nitrogen stream, and the resulting mixture was stirred for 2.5 hours. After the reaction was completed, a saturated aqueous solution of sodium hydrogen carbonate was added, the resulting mixture was subjected to extraction with ethyl acetate, washed with water and a saturated saline solution, and dried over anhydrous sodium sulfate, and then the solvent was distilled away under reduced pressure. Next, 1,2-dioxane (760 pL) and 28% aqueous ammonia (95 pL, 1.53 mmol) were added, and the resulting mixture was stirred at room temperature for 2.5 hours. After the reaction was completed, the reaction solution was distilled away under reduced pressure, and then the obtained residue was purified through silica gel column chromatography (SiO₂, containing 0.5% triethylamine, chloroform : methanol = 50 : 1) to obtain a compound 9 (46.4 mg, 93%) as a white solid. Table 8 shows data on the properties of the obtained compound 9.

**[Table 8]**

| Physical property data of the obtained compound 9 |
|---|
| ¹H NMR (300 MHz, CDCl₃) δ 0.53-0.64 (m, 6H), 0.67-0.85 (m, 1H), 0.94 (t, *J* = 8.5, 9H), 1.11-1.33 (m, 4H), 1.37 (s, 3H), 1.48-1.64 (m, 1H), 1.79-1.89 (m, 2H), 2.16-2.28 (m, 1H), 2.33-2.43 (m, 1H), 3.53 (d, *J* = 4.5 Hz, 1H), 4.87-4.96 (m, 1H), 6.43 (dd, *J* = 6.2, 7.6 Hz, 1H), 7.16-7.25 (m, 9H), 7.56-7.66 (m, 7H); ¹³C NMR (76 MHz, CDCl₃) δ 5.0, 7.0, 12.7, 23.6, 24.7, 33.9, 42.8, 71.1, 83.6, 89.7, 91.3, 101.9, 127.1, 127.5, 129.4, 138.9, 146.4, 156.1, 165.5; HRMS (MALDI): Calculated for C₃₉H₄₉N₃O₄NaSi [M+Na]⁺: 674.3388 Found: 674.3385. |

### (2-3) Synthesis of Compound 10

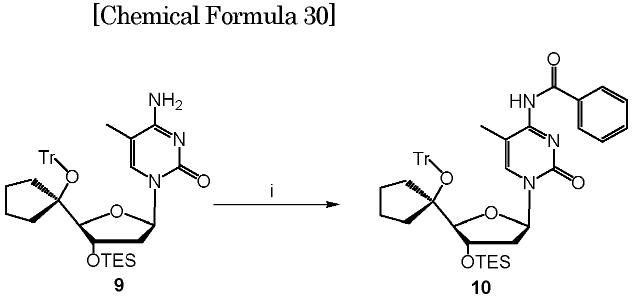

Anhydrous benzoyl (176 mg, 0.778 mmol) was added to a pyridine solution (5.1 mL) of the compound 9 obtained above (331 mg, 0.508 mmol) under a nitrogen stream, and the resulting mixture was stirred at room temperature for 14 hours. After the reaction was completed, a saturated aqueous solution of sodium hydrogen carbonate was added, the resulting mixture was subjected to extraction with ethyl acetate, washed with water and a saturated saline solution, and dried over anhydrous sodium sulfate, and then the solvent was distilled away under reduced pressure. The obtained residue was purified through silica gel column chromatography (SiO₂, containing 0.5% triethylamine, hexane : ethyl acetate = 11 : 1) to obtain a compound 10 (373 mg, 97%) as a white solid. Table 9 shows data on the properties of the obtained compound 10.

**[Table 9]**

| Physical property data of the obtained compound 10 |
|---|
| ¹H NMR (300 MHz, CDCl₃) δ 0.61 (q, *J* = 7.6 Hz, 6H), 0.67-0.89 (m, 1H), 0.96 (t, *J* = 8.3 Hz, 9H), 1.13-1.50 (m, 5H), 1.53 (s, 3H), 1.81-2.01 (m, 2H), 2.21-2.37 (m, 2H), 3.58 (d, *J* = 3.4 Hz, 1H), 4.92-4.98 (m, 1H), 6.41 (dd, *J* = 6.2 Hz, 1H), 7.20-7.31 (m, 7H), 7.39-7.55 (m, 3H), 7.56-7.67 (m, 10H), 8.29 (d, *J* = 8.2 Hz, 2H); ¹³C NMR (76 MHz, CDCl₃) δ 5.0, 6.9, 23.6, 24.7, 33.5, 33.9, 42.1, 71.2, 83.3, 85.9, 89.7, 92.0, 112.6, 127.2, 127.6, 128.2, 129.4, 130.0, 132.5, 137.3, 146.1, 148.2, 159.8, 179.7; HRMS (MALDI): Calculated for C₄₆H₅₃N₃O₅NaSi [M+Na]⁺: 778.3632 Found: 778.3647. |

### (2-4) Synthesis of Compound 11

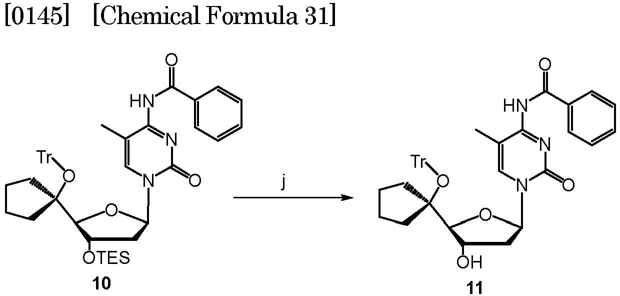

A 1.0 M tetrahydrofuran solution of tetrabutylammonium fluoride (208 pL, 0.208 mmol) was added to a tetrahydrofuran solution (2.4 mL) of the compound 10 obtained above (157 mg, 0.208 mmol), and the resulting mixture was stirred at room temperature for 1 hour. After the reaction was completed, the solvent was distilled away under reduced pressure, and then the obtained residue was purified through silica gel column chromatography (SiO₂, containing 0.5% triethylamine, hexane : ethyl acetate = 3 : 1) to obtain a compound 11 (105 mg, 79%) as a white solid. Table 10 shows data on the properties of the obtained compound 11.

**[Table 10]**

| Physical property data of the obtained compound 11 |
|---|
| ¹H NMR (500 MHz, CDCl₃) δ 1.01-1.11 (m, 1H), 1.18-1.58 (m, 5H), 1.75 (s, 3H), 1.82-2.11 (m, 1H), 1.95 (d, *J* = 3.5 Hz, 1H), 1.82-2.11 (m, 1H), 2.20-2.30 (m, 1H), 2.30-2.42 (m, 1H), 3.44 (d, *J* = 4.0 Hz 1H), 4.79-4.88 (m, 1H), 6.26 (dd, *J* = 6.3, 8.6 Hz, 1H), 7.16-7.34 (m, 10H), 7.40-7.45 (m, 3H), 7.45-7.65 (m, 7H), 8.29 (d, *J* = 7.5 Hz, 2H); ¹³C NMR (76 MHz, CDCl₃) δ 13.4,25.0,25.0,34.6,35.4,41.1,71.2,83.4,86.4,91.0,112.5,127.4,127.8,128.3,129.4,130.0, 132.2 132.6, 136.8, 137.2, 146.0, 148.2, 159.6, 179.7; HRMS (MALDI): Calculated for C₄₀H₃₉N₃O₅Na [M+Na]⁺: 664.2796 Found: 664.2782. |

### (2-5) Synthesis of Compound 12 (Amidite Block 12)

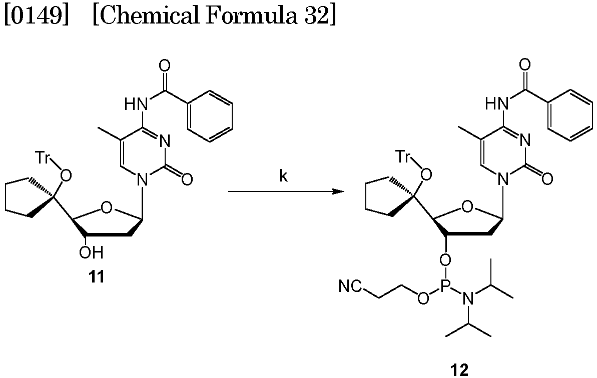

N,N-diisopropylethylamine (86 µL, 0.741 mmol) and 2-cyanoethyl-N,N-diisopropyl phosphorochloridate (82 µL, 0.378 mmol) were successively added to an anhydrous dichloromethane solution (1.6 mL) of the compound 11 obtained above (105 mg, 0.164 mmol) at 0°C under a nitrogen stream, and the resulting mixture was stirred at room temperature for 5.5 hours. After the reaction was completed, the solvent was distilled away under reduced pressure, and then the obtained residue was purified through silica gel column chromatography (SiO₂, containing 0.5% triethylamine, hexane : ethyl acetate = 3 : 1) to obtain a compound 12 (115 mg, 83%, diastereomer ratio 1 : 1) as a white solid. Table 11 shows data on the properties of the obtained compound 12.

**[Table 11]**

| Physical property data of the obtained compound 12 |
|---|
| ¹H NMR (500 MHz, CDCl₃) δ 0.80-1.01 (m, 1H), 1.10-1.60 (m, 21H), 1.72-1.95 (m, 2H), 2.28-2.44 (m, 1H), 2.52-2.58 (m, 1H), 2.63-2.70 (m, 1H), 3.53-3.93 (m, 5H), 4.99-5.08 (m, 1H), 6.39-6.46 (m, 1H), 7.22-7.31 (m, 10H), 7.39-7.47 (m, 2H), 7.48-7.57 (m, 2H), 7.58-7.64 (m, 6H), 8.29 (d, *J* = 7.8 Hz, 2H); ³¹P NMR (121.7 MHz, CDCl₃) δ 148.0, 148.8; HRMS (MALDI): Calculated for C₄₉H₅₆N₅O₆NaSi [M+Na]⁺: 864.3860 Found: 864.3887. |

### Example 3: Synthesis of 5'-Modified Nucleoside (3)

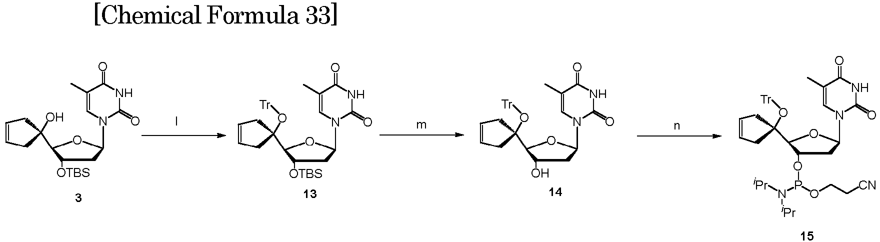

Reagent and conditions: (1) TrOTf, 2,6-lutidine, 80 °C, 22.5 h, 80%; (m) TBAF, THF, rt, 22.5 h, 90%.; (n) iPr₂NP(Cl)OCH₂CH₂CN, DIPEA, MeCN, rt, 1.25 h, 76%.

### (3-1) Synthesis of Compound 13

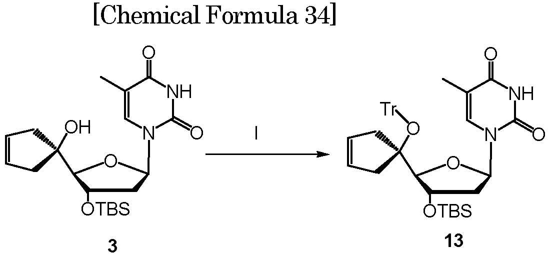

Silver trifluoromethanesulfonate (1152 mg, 4.48 mmol) was added to an anhydrous dichloromethane solution (7.3 mL) of trityl chloride (1254 mg, 4.50 mmol) and mixed. The orange suspension was stirred at room temperature for 1.5 hours, and then silver chloride was precipitated. The supernatant, a trityl triflate solution, (3.6 mL) was added to an anhydrous 2,6-lutidine solution (3.6 mL) of the compound 3 obtained above (300 mg, 0.734 mmol) at 0°C. After the reaction solution was stirred at 80°C for 22.5 hours, a saturated aqueous solution of sodium hydrogen carbonate was added to the reaction solution. After a saturated aqueous solution of copper sulfate was further added to emulsify lutidine, the resulting mixture was filtered through cerite using ethyl acetate. The solvent was distilled away under reduced pressure, and then the obtained residue was purified through silica gel column chromatography (SiO₂, containing 0.5% triethylamine, hexane : ethyl acetate = 5 : 1 → 7 : 3) to obtain a compound 13 (382 mg, 80%) as a yellow solid. Table 12 shows data on the properties of the obtained compound 13.

**[Table 12]**

| Physical property data of the obtained compound 13 |
|---|
| ¹H NMR (500 MHz, CDCl₃) δ -0.01 (s, 3H), 0.08 (s, 3H), 0.86 (s, 9H), 1.54 (bs, 3H), 2.11-2.34 (m, 5H), 2.64 (d, *J* = 18 Hz, 1H), 3.30-3.57 (m, 1H), 4.40--4.77 (m, 1H), 5.21-5.42 (m, 2H), 6.17-6.38 (m, 2H), 7.22-7.26 (m, 8H), 7.39 (s, 1H), 7.48-7.59 (m, 7H), 8.56 (s, 1H); ¹³C NMR (126 MHz, CDCl₃) δ -4.9, -3.9, 12.3, 17.8, 25.8, 40.1, 41.7, 71.4, 83.0, 86.3, 87.5, 92.3, 111.3, 127.2, 127.7, 128.6, 128.8, 129.3, 135.9, 145.9, 150.6, 163.9; HRMS (MALDI): Calculated for C₃₉H₄₆N₂O₅NaSi [M+Na]⁺: 673.3038 Found: 673.3068. |

### (3-2) Synthesis of Compound 14

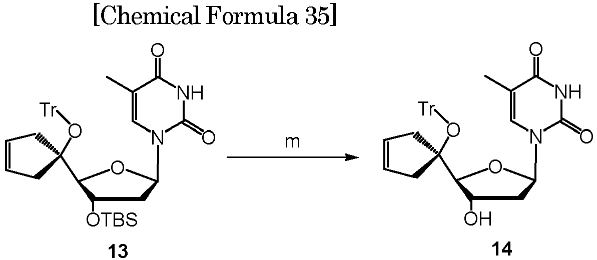

A 1.0 M tetrahydrofuran solution of tetrabutylammonium fluoride (335 pL, 0.335 mmol) was added to a tetrahydrofuran solution (5.5 mL) of the compound 13 obtained above (218 mg, 0.335 mmol), and the resulting mixture was stirred at room temperature for 19 hours. After the reaction was completed, the solvent was distilled away under reduced pressure, and then the obtained residue was purified through silica gel column chromatography (SiO₂, containing 0.5% triethylamine, chloroform : methanol = 15 : 1) to obtain a compound 14 (180 mg, quantitative) as a white solid. Table 13 shows data on the properties of the obtained compound 14.

**[Table 13]**

| Physical property data of the obtained compound 14 |
|---|
| ¹H NMR (500 MHz, CDCl₃) δ 1.54 (s, 3H), 2.08-2.49 (m, 6H), 2.61-2.74 (m, 1H), 3.51 (d, *J* = 1.7 Hz, 1H), 4.70--4.80 (m, 1H), 5.24-5.32 (m, 1H), 5.33-5.40 (m, 1H), 6.31 (dd, *J* = 6.3, 6.3 Hz, 1H), 7.18-7.32 (m, 10H), 7.44-7.54 (m, 6H), 8.86 (bs, 1H); ¹³C NMR (76 MHz, CDCl₃) δ 12.3, 40.6, 41.2, 71.4, 83.3, 86.6, 87.6, 92.1, 111.4, 127.3, 127.9, 128.1, 129.1, 129.3, 135.7, 145.8, 150.6, 163.7; HRMS (MALDI): Calculated for C₃₃H₃₂N₂O₅Na [M+Na]⁺: 559.2205 Found: 559.2203. |

### (3-3) Synthesis of Compound 15 (Amidite Block 15)

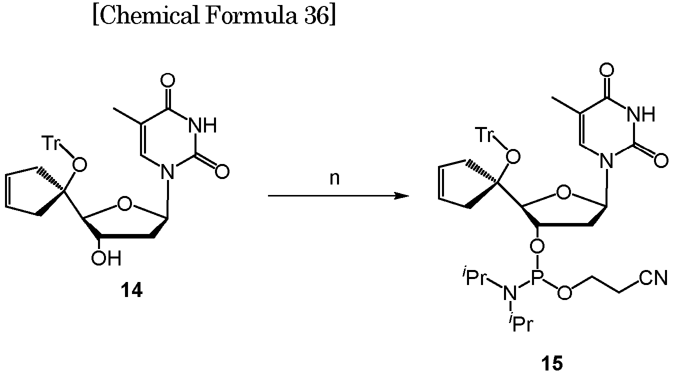

N,N-diisopropylethylamine (290 pL, 1.66 mmol) and 2-cyanoethyl-N,N-diisopropyl phosphorochloridate (190 µL, 0.852 mmol) were successively added to an anhydrous acetonitrile solution (5.6 mL) of the compound 14 obtained above (300 mg, 0.559 mmol) at 0°C under a nitrogen stream, and the resulting mixture was stirred at room temperature for 1.25 hours. After the reaction was completed, the solvent was distilled away under reduced pressure, and then the obtained residue was purified through silica gel column chromatography (SiO₂, containing 0.5% triethylamine, hexane : ethyl acetate = 2 : 1). Furthermore, reprecipitation (toluene/hexane) was conducted to obtain a compound 15 (306 mg, 76%, diastereomer ratio 1 : 1) as a white solid. Table 14 shows data on the properties of the obtained compound 15.

**[Table 14]**

| Physical property data of the obtained compound 15 |
|---|
| ¹H NMR (500 MHz, CDCl₃) δ 0.79-0.91 (m, 1H), 1.09-1.28 (m, 14H), 1.36-1.50 (m, 2H), 2.07-2.72 (m, 7H), 3.52-3.91 (m, 5H), 4.75-4.95 (m, 1H), 5.19-5.38 (m, 2H), 6.28-6.45 (m, 1H), 7.20-7.28 (m, 8H), 7.29-7.32 (m, 1H), 7.46-7.45 (m, 6H), 8.73 (bs, 1H); ³¹P NMR (121.7 MHz, CDCl₃) δ 147.9, 148.8; HRMS (MALDI): Calculated for C₄₂H₄₉N₄O₆Na [M+Na]⁺: 759.3273 Found: 759.3281. |

### Example 4: Synthesis of 5'-Modified Nucleoside (4)

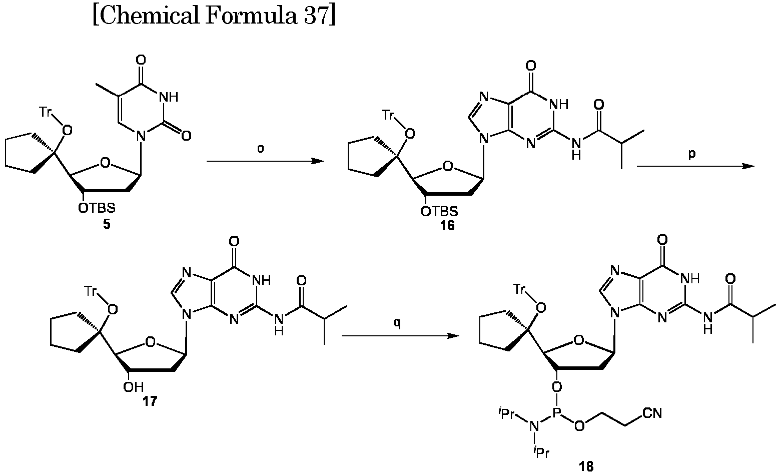

Reagents and conditions: (o) *N²*-isobutyrylguanine, TMSOTf, BSA, toluene, 80 °C, 2 h, 39%; (p) TBAF, THF, 50 °C, 6.5h, 81%; (q) iPr₂NP(Cl)OCH₂CH₂CN, DIPEA, DCM, rt, 5 h, 52%.

### (4-1) Synthesis of Compound 16

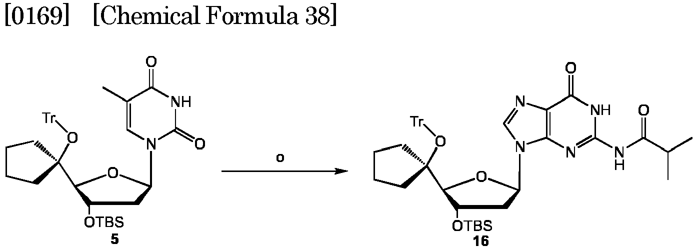

After N²-isobutyrylguanine (381 mg, 1.73 mmol) and N,O-bis(trimethylsilyl)acetamide (2.0 mL, 8.05 mmol) were added to an anhydrous toluene solution (11 mL) of the compound 5 obtained above (750.7 mg, 1.15 mmol) under a nitrogen stream, trimethylsilyl trifluoromethanate (21 µL, 0.115 mmol) was added at 0°C, and the resulting mixture was stirred at 80°C for 2 hours. After the reaction was completed, a saturated aqueous solution of sodium hydrogen carbonate was added, and insoluble matter was removed through cerite filtration. After the filtrate was subjected to extraction with ethyl acetate, the extraction fraction was washed with water and a saturated saline solution and then dried over anhydrous sodium sulfate, and the solvent was distilled away under reduced pressure. The obtained residue was purified through silica gel column chromatography (SiO₂, containing 0.5% triethylamine, chloroform : methanol = 300 : 1) to obtain a compound 16 (332 mg, 39%) as a white solid. Table 15 shows data on the properties of the obtained compound 16.

**[Table 15]**

| Physical property data of the obtained compound 16 |
|---|
| ¹H NMR (500 MHz, CDCl₃) δ 0.06 (s, 3H), 0.14 (s, 3H), 0.34 (d, *J* = 7.0 Hz, 3H), 0.43-0.52 (m, 1H), 0.77 (d, *J* = 7.0 Hz, 3H), 0.90 (s, 9H), 1.04-1.32 (m, 5H), 1.35-1.41 (m, 1H), 1.82-1.88 (m, 2H), 2.25 (dd, *J=* 4.5, 12.5 Hz, 1H), 3.51-3.60 (m, 1H), 3.67 (d, *J* = 3.0 Hz, 1H), 5.14 (dd, *J* = 3.0, 7.0 Hz, 1H), 6.05 (dd, *J* = 5.0,10.3 Hz, 1H), 6.85 (s, 1H), 7.16-7.20 (m, 8H), 7.54-7.58 (m, 1H), 7.69-7.74 (m, 6H), 7.83 (s, 1H), 11.89 (bs, 1H); ¹³C NMR (76 MHz, CDCl₃) δ -4.8, -3.9, 17.9, 18.5, 18.6, 23.0, 24.4, 25.9, 32.7, 34.2, 35.8, 40.6, 71.4, 84.9, 85.5, 89.6, 93.0, 127.2, 127.5, 127.6, 129.5, 129.7, 139.7, 146.7, 147.0, 155.7, 178.1; HRMS (MALDI): Calculated for C₄₃H₅₃N₅O₅NaSi [M+Na]⁺: 770.3706, Found: 770.3708. |

### (4-2) Synthesis of Compound 17

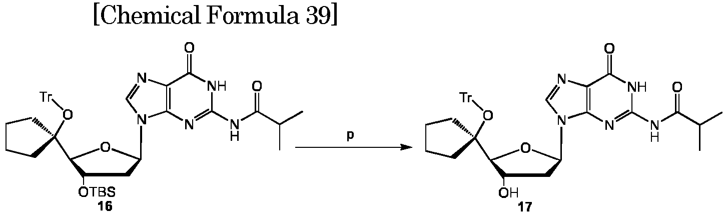

A 1.0 M tetrahydrofuran solution of tetrabutylammonium fluoride (609 pL, 0.609 mmol) was added to a tetrahydrofuran solution (7.0 mL) of the compound 16 obtained above (456 mg, 0.609 mmol), and the resulting mixture was stirred at 50°C for 6.5 hours. After the reaction was completed, the solvent was distilled away under reduced pressure, and then the obtained residue was purified through silica gel column chromatography (Diol Silica, containing 1.0% triethylamine, chloroform : methanol = 200 : 1) to obtain a compound 17 (367.3 mg, 95%) as a white solid. Table 16 shows data on the properties of the obtained compound 17.

**[Table 16]**

| Physical property data of the obtained compound 17 |
|---|
| ¹H NMR (300 MHz, CDCl₃) δ 0.64 (d, *J* = 6.6 Hz, 3H), 0.91 (d, *J* = 6.9 Hz, 3H), 1.07-1.51 (m, 7H), 1.62-1.74 (m, 1H), 1.82-1.92 (m, 2H), 2.41-2.47 (m, 1H), 3.24-3.36 (m, 1H), 3.67 (d. *J* = 3.3 Hz, 1H), 5.08-5.13 (m, 1H), 6.19 (dd, *J* =4.8, 9.3 Hz, 1H), 7.16-7.22 (m, 9H), 7.63-7.69 (m, 6H), 7.88 (bs, 2H), 12.01 (s, 1H); ¹³C NMR (76 MHz, CDCl₃) δ 18.7, 18.8, 24.0, 24.7, 33.8, 34.5, 36.0, 40.6, 71.1, 84.3, 85.8, 89.8, 92.1, 122.3, 127.2, 127.6, 128.1, 129.6, 139.2, 146.7, 147.2, 155.8, 178.8; HRMS (MALDI): Calculated for C₃₇H₃₉N₅O₅Na [M+Na]⁺: 656.2847, Found: 656.2843. |

### (4-3) Synthesis of Compound 18 (Amidite Block 18)

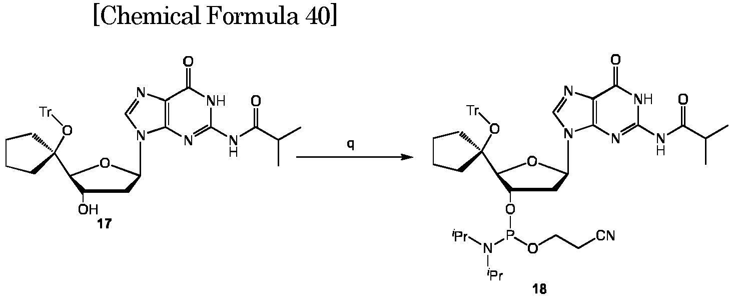

N,N-diisopropylethylamine (42 pL, 0.238 mmol) and 2-cyanoethyl-N,N-diisopropyl phosphorochloridate (27 µL, 0.119 mmol) were successively added to an anhydrous dichloromethane solution (794 µL) of the compound 17 obtained above (50.3 mg, 0.0794 mmol) at 0°C under a nitrogen stream, and the resulting mixture was stirred at room temperature for 5 hours. After the reaction was completed, the solvent was distilled away under reduced pressure, and then the obtained residue was purified through silica gel column chromatography (Diol Silica, containing 1.0% triethylamine, hexane : ethyl acetate = 1 : 1). Furthermore, reprecipitation (chloroform/ethyl acetate) was conducted to obtain a compound 18 (34.4 mg, 52%, diastereomer ratio 1 : 1) as a white solid. Table 17 shows data on the properties of the obtained compound 18.

**[Table 17]**

| Physical property data of the obtained compound 18 |
|---|
| ¹H NMR (300 MHz, CDCl₃) δ 0.38 (d, *J* = 6.9 Hz, 1.5H), 0.56 (d, *J* = 6.9 Hz, 1.5H), 0.78 (d,*J* = 6.6 Hz, 1.5H), 0.88 (d, *J* = 6.3 Hz, 1.5H), 1.07-1.35 (m, 18H), 1.39-1.48 (m, 2H), 1.81-1.93 (m, 2H), 2.47-2.87 (m, 3H), 3.56-3.95 (m, 5H), 5.11-5.30 (m, 1H), 5.96-6.09 (m, 1H), 7.15-7.22 (m, 9H), 7.61-7.73 (m, 6H), 7.80 (s, 0.5H), 7.89 (s, 0.5H), 11.83-11.90 (m, 1H); ³¹P NMR (121.7 MHz, CDCl₃) δ 148.2, 149.3; HRMS (MALDI): Calculated for C₄₆H₅₆N₇O₆NaP [M+Na]⁺: 856.3922, Found: 856.3931. |

### Example 5: Synthesis of 5'-Modified Nucleoside (5)

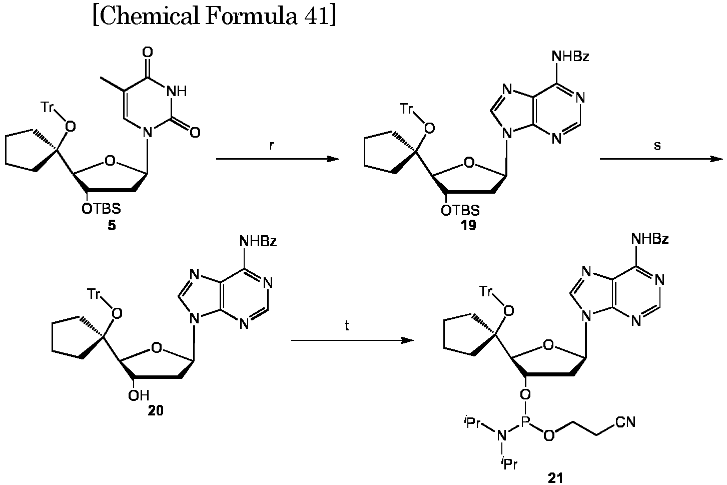

**Reagents and conditions:** (r) *N⁶*-benzoyladenine, TMSOTf, BSA, toluene, 80 °C, 1.5 h, 28%; (s) TBAF, THF, 50 °C, 3.5 h, quant.; (t) iPr₂NP(Cl)OCH₂CH₂CN, DIPEA, DCM, rt, 5 h, 51%.

### (5-1) Synthesis of Compound 19

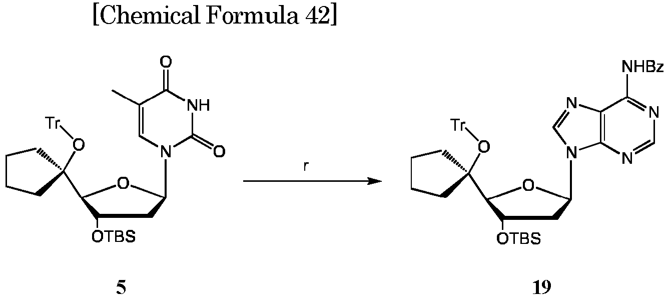

After N⁶-benzoyladenine (501 mg, 2.10 mmol) and N,O-bis(trimethylsilyl)acetamide (2.1 mL, 8.38 mmol) were added to an anhydrous toluene solution (14 mL) of the compound 5 obtained above (912.4 mg, 1.40 mmol) under a nitrogen stream, trimethylsilyl trifluoromethanate (25 pL, 0.140 mmol) was added at 0°C, and the resulting mixture was stirred at 80°C for 1.5 hours. After the reaction was completed, a saturated aqueous solution of sodium hydrogen carbonate was added, and insoluble matter was removed through cerite filtration. After the filtrate was subjected to extraction with ethyl acetate, the extraction fraction was washed with water and a saturated saline solution and then dried over anhydrous sodium sulfate, and the solvent was distilled away under reduced pressure. The obtained residue was purified through silica gel column chromatography (SiO₂, containing 0.5% triethylamine, chloroform) to obtain a compound 19 (303 mg, 28%) as a white solid. Table 18 shows data on the properties of the obtained compound 19.

**[Table 18]**

| Physical property data of the obtained compound 19 |
|---|
| ¹H NMR (500 MHz, CDCl₃) δ 0.06 (s, 3H), 0.14 (s, 3H), 0.90 (s, 9H), 1.18-1.44 (m, 5H), 1.50-1.57 (m, 1H), 1.75-1.88 (m, 2H), 2.43-2.49 (m, 1H), 2.87-2.94 (m, 1H), 5.80 (d, *J* = 3.5 Hz, 1H), 4.93-4.93 (m, 1H), 6.41 (dd, *J =* 5.8, 8.8Hz, 1H), 7.17-7.21 (m, 8H), 7.50-7.63 (m, 10H), 8.03 (d, *J =* 7.5 Hz, 2H), 8.23 (s, 1H), 8.83 (s, 1H), 9.02 (bs, 1H); ¹³C NMR (126 MHz, CDCl₃) δ -4.7, -3.9, 17.9,24.0, 24.9,25.9, 33.2,34.9, 42.1,71.6, 82.8, 85.7,89.7, 92.4, 123.2, 126.6, 127.5, 128.0, 128.1, 129.0, 132.9, 141.5,146.3,151.9,153.1,164.6; HRMS (MALDI): Calculated for C₄₆H₅₁N₅O₄SiNa [M+Na]⁺: 788.3600, Found: 788.3603. |

### (5-2) Synthesis of Compound 20

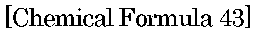

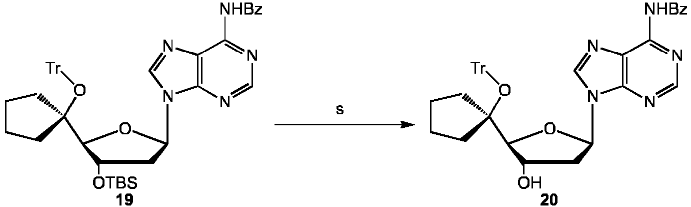

A 1.0 M tetrahydrofuran solution of tetrabutylammonium fluoride (199 pL, 0.199 mmol) was added to a tetrahydrofuran solution (2.3 mL) of the compound 19 obtained above (153 mg, 0.199 mmol), and the resulting mixture was stirred at 50°C for 3.5 hours. After the reaction was completed, the solvent was distilled away under reduced pressure, and then the obtained residue was purified through silica gel column chromatography (Diol Silica, containing 1.0% triethylamine, hexane : ethyl acetate = 1 : 1 → chloroform) to obtain a compound 20 (130 mg, quantitative) as a white solid. Table 19 shows data on the properties of the obtained compound 20.

**[Table 19]**

| Physical property data of the obtained compound 20 |
|---|
| ¹H NMR (500 MHz, CDCl₃) δ 1.13-1.48 (m, 6H), 1.95-2.09 (m, 2H), 2.28 (bs, 1H), 2.46-2.51 (m, 1H), 2.84-2.91 (m, 1H), 3.47 (d, *J* = 4.5 Hz, 1H), 4.93-4.97 (m, 1H), 6.28 (dd,*J* = 7.0, 7.0 Hz, 1H), 7.21-7.30 (m, 8H), 7.49-7.62 (m, 10H), 8.02 (d, *J* = 7.5 Hz, 2H), 8.09 (s, 1H), 8.78 (bs, 1H), 9.10 (bs, 1H); ¹³C NMR (126 MHz, CDCl₃) δ 25.1, 26.0, 35.5, 36.1, 40.5, 71.4,83.0,86.6,90.2,91.0,123.4,127.3,127.9,128.0,129.0,129.3,133.0,141.4,146.0,149.6, 151.8, 152.9, 164.7; HRMS (MALDI): Calculated for C₄₀H₃₇N₅O₄Na [M+Na]⁺: 674.2738, Found: 674.2738. |

### (5-3) Synthesis of Compound 21 (Amidite Block 21)

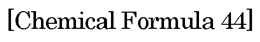

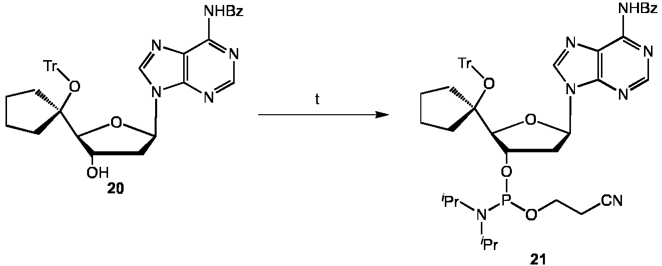

N,N-diisopropylethylamine (41 pL, 0.233 mmol) and 2-cyanoethyl-N,N-diisopropyl phosphorochloridate (26 µL, 0.117 mmol) were successively added to an anhydrous dichloromethane solution (778 µL) of the compound 20 obtained above (50.7 mg, 0.0778 mmol) at 0°C under a nitrogen stream, and the resulting mixture was stirred at room temperature for 5 hours. After the reaction was completed, water was added, the resulting mixture was subjected to extraction with ethyl acetate, the extraction fraction was washed with water and a saturated saline solution and then dried over anhydrous sodium sulfate, and the solvent was distilled away under reduced pressure. The obtained residue was purified through silica gel column chromatography (Diol Silica, containing 1.0% triethylamine, hexane : ethyl acetate = 3 : 2). Furthermore, reprecipitation (ethyl acetate/hexane) was conducted to obtain a compound 21 (34.0 mg, 51%, diastereomer ratio 3 : 2) as a white solid. Table 20 shows data on the properties of the obtained compound 21.

**[Table 20]**

| Physical property data of the obtained compound 21 |
|---|
| ¹H NMR (300 MHz, CDCl₃) δ 1.13-1.31 (m, 17H), 1.44-1.49 (m, 1H), 1.80-1.89 (m, 2H), 2.54 (dd, *J* = 6.3, 6.3 Hz, 1H), 2.66 (dd, *J* = 6.0, 6.0 Hz, 1H), 2.69-3.02 (m, 2H), 3.56-3.95 (m, 5H), 5.07-5.18 (m 1H), 6.44-6.50 (m, 1H), 7.18-7.23 (m, 8H), 7.49-7.62 (m, 10H), 8.01-8.05 (m, 2H), 8.17 (s, 0.6H), 8.20 (s, 0.4H), 8.80 (s, 0.6H), 8.84 (s, 0.4H), 9.03 (bs, 0.6H), 9.07 (bs, 0.4H); ³¹P NMR (121.7 MHz, CDCl₃) δ 148.7, 149.7; HRMS (MALDI): Calculated for C₄₉H₅₄N₇O₅NaP [M+Na]⁺: 874.3837, Found: 874.3816. |

### (Example 6) Synthesis and Purification of Oligonucleotide

0.1 M anhydrous dichloromethane solutions of the amidite blocks 7, 15, 18, and 21 obtained above were prepared for synthesis of oligonucleotides for use in Examples below, and the oligonucleotide synthesis was performed trityl-on using nS-8 Oligonucleotides Synthesizer (manufactured by GeneDesign, Inc.). Activator-42 (registered trademark) (0.25 M acetonitrile solution) was used as an activator, and the condensation time was extended to 240 seconds × 4 for the amidite blocks 7, 15, 18, and 21 and amidites to be introduced next. The capping time was 200 seconds. Also, the deblocking time was extended to 150 seconds × 2. With regard to the other operations, the synthesis was performed according to an ordinary phosphoramidite method known per se. After the synthesis was completed, the product was treated with a 23% aqueous solution of ammonia at room temperature for 4 hours, thus cleaved from the column support, and subsequently allowed to stand at 55°C for 5 hours or more for deprotection. Subsequently, the trityl group was removed using a simple reverse-phase column (Sep-Pak (registered trademark) Plus C18 Environmental Cartridges manufactured by Waters Corporation) to purify the oligonucleotide, and then the oligonucleotide was further purified through reverse-phase HPLC.

### (Example 7) Composition Determination and Quantification

A matrix (10 mg/mL aqueous solution of 3-hydroxypicolinic acid : 1 mg/mL aqueous solution of diammonium citrate = 1 : 1, 1 pL) was dried on an AnchorChip, and an aqueous solution of the oligonucleotide (50 µM, 1 pL) was placed on the AnchorChip and then dried again. After that, the composition of the oligonucleotide was determined through MALDI-TOF MS. The molecular weight was measured in a negative mode, and oligothymidylic acids (7 mer, 9 mer, 11 mer, and 13 mer) were used as external standards. The oligonucleotide was quantified by measuring ultraviolet absorption at 260 nm using an absorbance measurement apparatus.

### (Example 8) Measurement of Melting Temperature (Tₘ)

A mixed solution of each oligonucleotide (final concentration: 4 µM) and a phosphate buffer (10 mM, pH 7.2, 130 pL) containing sodium chloride (final concentration: 100 mM) was bathed in boiled water and then slowly cooled to room temperature. Thereafter, the solution was cooled to 5°C under a nitrogen stream before starting the measurement. The temperature was raised to 90°C at a rate of 0.5°C/minute while absorbance at 260 nm was plotted at intervals of 0.5°C. The Tₘ value was calculated using a median method, and a mean value of three independent measurements was adopted. Table 21 shows the results. In (I) in Table 21, the sequence of the ssRNA is 5'-r(AGCAAAAAACGC)-3' (SEQ ID NO: 19), and the right superscripts 1 to 3 indicate 5'-cp, 5'-cp2, and 5'-cp3, respectively. In (II) in Table 21, the sequence of the ssRNA is 5'-r(AGCAAAGAACGC)-3' (SEQ ID NO: 26), and ^{m}C^{cp3} indicates 5'-cp3-5-methylcytosine. In (III) in Table 21, the sequence of the ssRNA is 5'-r(AGCAAAUAACGC)-3' (SEQ ID NO: 27), and A^{cp3} indicates 5'-cp3-adenine. In (IV) in Table 21, the sequence of the ssRNAis 5'-r(AGCAAACAACGC)-3' (SEQ ID NO: 28), and G^{cp3} indicates 5'-cp3-guanine.

**[Table 21]**

| | Sequence (5' -3' ) | Toward ssRNA | Toward ssDNA | | | |
|---|---|---|---|---|---|---|
| | | *Tₘ*(°C) | Δ*Tₘ*(°C) | *Tₘ*(°C) | Δ*Tₘ*(°C) | SEQ ID NO. |
| (I) | GCGTTTTTTGCT | 47 | - | 52 | - | SEQ ID NO: 1 |
| | GCGTTT¹TTTGCT | 44 | -3 | 48 | -4 | SEQ ID NO: 2 |
| | GCGTTT²TTTGCT | 45 | -2 | 49 | -3 | SEQ ID NO: 3 |
| | GCGTTT³TTTGCT | 46 | -1 | 49 | -3 | SEQ ID NO: 4 |
| | GCGTT³TT³TT³GCT | 44 | -3 | 46 | -6 | SEQ ID NO: 5 |
| | GCGTT³T³T³TTGCT | 45 | -2 | 47 | -5 | SEQ ID NO: 6 |
| (II) | GCGTTCTTTGCT | 53 | - | 53 | - | SEQ ID NO: 20 |
| | GCGTT^{m}C^{cp3}TTTGCT | 53 | 0 | 54 | +1 | SEQ ID NO: 21 |
| (III) | GCGTTATITGCT | 46 | - | 50 | - | SEQ ID NO: 22 |
| | GCGTTA^{cp3}TTTGCT | 46 | 0 | 49 | -1 | SEQ ID NO: 23 |
| (IV) | GCGTTGTITGCT | 52 | - | 54 | - | SEQ ID NO: 24 |
| | GCGTTG^{cp3}TTTGCT | 52 | 0 | 55 | +1 | SEQ ID NO: 25 |

| | | | | | | |
|---|---|---|---|---|---|---|
| T¹: 5'-cp-thymidine, T²: 5'-cp2- thymidine, T³: 5'-cp3- thymidine, ^{m}C^{cp3}: 5'-cp3-5- thymidine, A^{cp3}: 5'-cp3-adenine, G^{cp3}: 5'-cp3-guanine | | | | | | |

As can be seen from Table 21, the Tₘ values of the oligonucleotides containing the artificial nucleic acids of the present invention represented by SEQ ID NOs: 3 and 4 were higher than the Tₘ value of the oligonucleotide containing the conventional artificial nucleic acid represented by SEQ ID NO: 2. It is found from this result that the double-strand forming ability for RNA of the artificial nucleic acids with the 5'-cp2 and the 5'-cp3 of the present invention is higher and better than that of the artificial nucleic acid with conventional 5'-cp.

### (Example 9) Evaluation of Enzyme Resistance

3'-Exonuclease (snake venom phosphodiesterase, svPDE) was added, at a concentration of 1.5 pg/ mL, to a 50 mM tris-hydrochloric acid buffer (pH 8.0) containing 7.5 pM oligonucleotide and 10 mM magnesium chloride, and the mixture was incubated at 37°C. 0, 2.5, 5.0, 10, 20, and 40 minutes after the start of the incubation, a10-µL aliquots were taken from the specimen and analyzed through reverse-phase HPLC to calculate the percentages of uncleaved oligonucleotides. The evaluation was derived from three independent measurements. Table 22 shows the oligonucleotides used for this evaluation.

**[Table 22]**

| Sequence (5' -3' ) | SEQ ID NO |
|---|---|
| TTTTTTTTTT | SEQ ID NO: 7 (Natural (PO)) |
| TTTTTTTTT^T | SEQ ID NO: 8 (PS) |
| TTTTTTTTTT¹ | SEQ ID NO: 9 (5'-cp(cp)) |
| TTTTTTTTTT² | SEQ ID NO: 10 (5'-cp2(cp2nd)) |
| TTTTTTTTTT³ | SEQ ID NO: 11 (5'-cp3(cp3rd)) |

| | |
|---|---|
| T¹: 5'-cp--thymidine, T₂: 5'-cp2--thymidine, T³: 5'-cp3--thymidine, ^: phosphorothioate (PS) | |

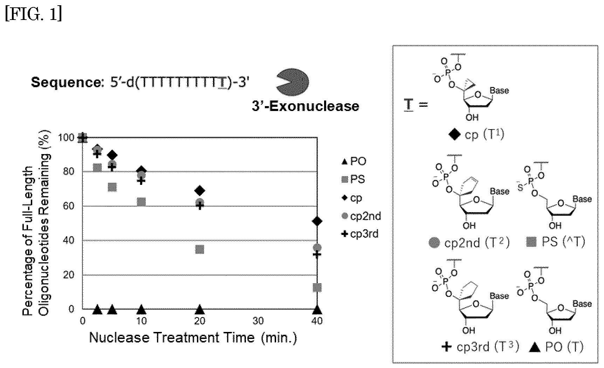

It was found from this experiment that the stability of the oligonucleotides containing the artificial nucleic acids of the present invention represented by SEQ ID NOs: 10 and 11 against 3'-exonuclease was higher than that of the oligonucleotide containing a phosphorothioate (PS)-modified nucleic acid represented by SEQ ID NO: 8, which is widely used in nucleic acid drugs (FIG. 1).

### (Example 10) Animal Study

Test oligonucleotides (100 nmol) were administered to the caudal veins of 6-week-old mice (C57BL/6NCrl, male: purchased from The Jackson Laboratory Japan, Inc.) (n=5). A physiological saline solution was administered as a control. After 96 hours, blood was collected under inhalation anesthesia (isoflurane), and the mice were exsanguinated. Thereafter, the organs were collected, the weight of the liver was measured, and RNA was extracted (RNeasy Mini Kit: manufactured by QIAGEN K.K.). The activities of aspartate transaminase (AST) and alanine transaminase (ALT) in the blood were measured using an automated analyzer (TBA-2000FR manufactured by CANON MEDICAL SYSTEMS CORPORATION). The mRNA expression levels of the target gene GR (NR3C 1) and the house keeping gene GAPDH were measured through real-time PCR (kit used: One Step PrimeScript RTPCR Kit (manufactured by Takara Bio Inc.), primer sequences: NR3C 1 forward (actgtccagcatgccgctat) (SEQ ID NO: 12), NR3C1 reverse (gcagtggcttgctgaattcc) (SEQ ID NO: 13), GAPDH forward (gtgtgaacggatttggccgt) (SEQ ID NO: 14), and GAPDH reverse (gacaagcttcccattctcgg) (SEQ ID NO: 15)).

**[Table 23]**

| Test oligonucleotides : | |
|---|---|
| ID | Sequence (5' -3' ) |
| Posi12 (SEQ ID NO: 16) | ^{L}G^^{L}T^^{L}C^t^c^t^t^t^a^c^c^^{L}T^^{L}G^^{L}G |
| Posi12-4cp3 (SEQ ID NO: 17) | ^{L}G^^{L}T^^{L}C**T**^c^t^t^t^a^c^c^^{L}T^^{L}G^^{L}G |
| Posi12-7cp3 (SEQ ID NO: 18) | ^{L}G^^{L}T^^{L}C^t^c^t**T**^t^a^c^c^^{L}T^^{L}G^^{L}G |

| | |
|---|---|
| **T**: 5' -cp3-T, ^{L}G: LNA-G, ^{L}C: LNA-^{m}C, ^{L}T: LNA-T, ^: Phosphorothioate (PS) | |

### (Results from Analysis of Target Gene Suppressing Effect)

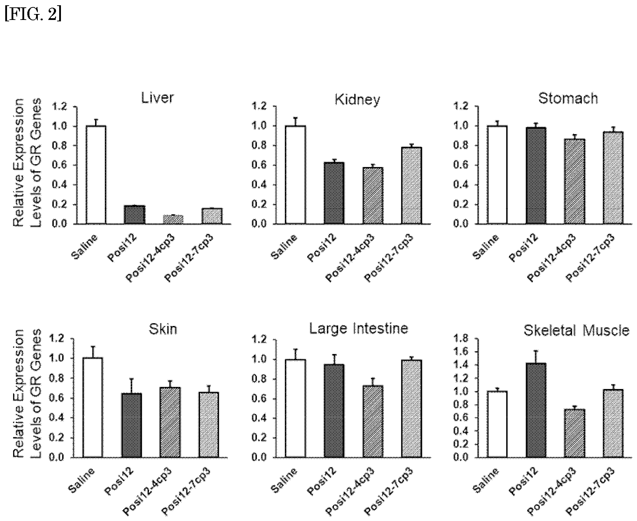

It was found from the results from this study that the antisense effect of the oligonucleotides containing the artificial nucleic acids of the present invention represented by SEQ ID NOs: 17 and 18 was higher than or equal to that of the oligonucleotide containing a phosphorothioate (PS)-modified nucleic acid represented by SEQ ID NO: 16, which is widely used in nucleic acid drugs, and is usable as an alternative to the widely used PS modification (FIG. 2). Also, it was found that these oligonucleotides can exhibit a better antisense effect in the liver, the large intestine, and the skeletal muscle, compared with the widely used PS modification (FIG. 2).

### (Evaluation Results of AST/ALT)

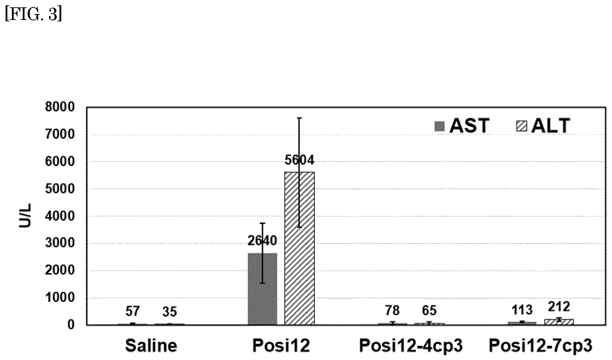

It was found that, when the oligonucleotides containing the artificial nucleic acids of the present invention represented by SEQ ID NOs: 17 and 18 were used, AST (U/L) and ALT (U/L), which are hepatotoxicity indicators, significantly decreased compared with the case of using the oligonucleotide containing a phosphorothioate (PS)-modified nucleic acid represented by SEQ ID NO: 16, which is widely used in nucleic acid drugs (FIG. 3). It can be understood from this result that the artificial nucleic acid of the present invention and the oligonucleotide containing the artificial nucleic acid can exhibit desired antisense effect while suppressing a toxicity to living organisms compared with oligonucleotides containing a phosphorothioate (PS)-modified nucleic acid, which are widely used in pharmaceuticals.

### Industrial Applicability

An oligonucleotide obtained using the 5'-modified nucleoside of the present invention is useful as, for example, a material for nucleic acid drugs.

## Claims

1. A compound represented by a formula (I) below or a salt thereof 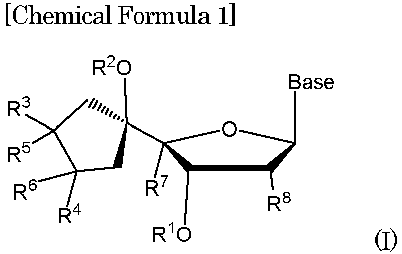 (in the formula (I),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the a group and may include a heteroatom, an aralkyl group with an aryl moiety having 3 to 12 carbon atoms that may have any one or more substituents selected from the a group and may include a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

2. The compound or salt thereof according to claim 1, wherein the formula (I) is represented by a formula (Ia) below: 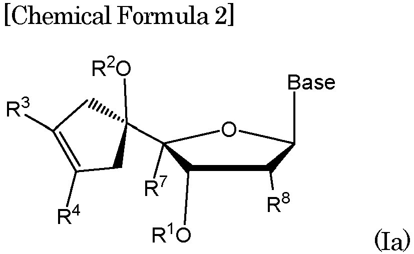 (in the formula (Ia),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the a group and may include a heteroatom, an aralkyl group with an aryl moiety having 3 to 12 carbon atoms that may have any one or more substituents selected from the a group and may include a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

3. The compound or salt thereof according to claim 1, wherein the formula (I) is represented by a formula (Ib) below: 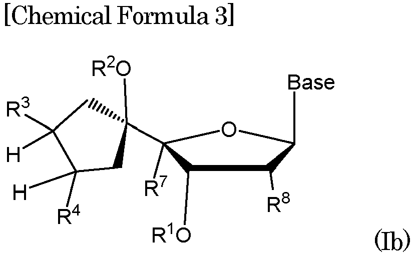 (in the formula (Ib),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the a group and may include a heteroatom, an aralkyl group with an aryl moiety having 3 to 12 carbon atoms that may have any one or more substituents selected from the a group and may include a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

4. The compound or salt thereof according to claim 1, wherein the Base in the formula (I) is a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-ffuoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, or a 4-amino-5-methyl-2-oxo-1,2-dihydropyrimidin-1-yl group.

5. The compound or salt thereof according to claim 1, wherein the Base in the formula (I) is a group represented by a formula below:

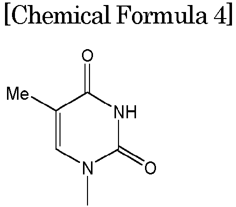

6. An oligonucleotide containing at least one nucleoside structure represented by a formula (II) below or a pharmacologically acceptable salt thereof 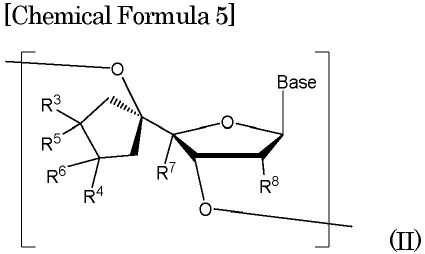 (in the formula (II),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

7. The oligonucleotide or pharmacologically acceptable salt thereof according to claim 6, wherein the formula (II) is represented by a formula (IIa) below: 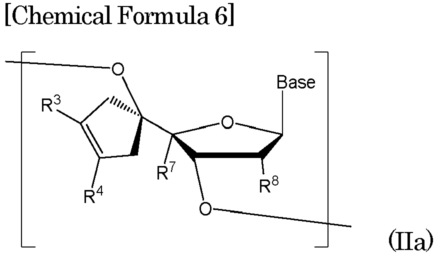 (in the formula (IIa),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

8. The oligonucleotide or pharmacologically acceptable salt thereof according to claim 6, wherein the formula (II) is represented by a formula (IIb) below: 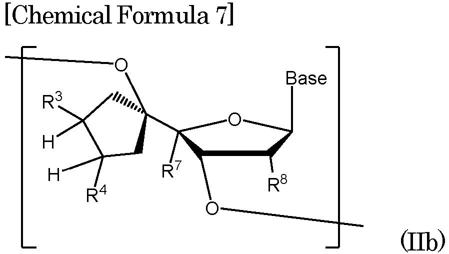 (in the formula (IIb),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).

9. A method for producing the oligonucleotide or pharmacologically acceptable salt thereof according to claim 6, comprising a step of producing an oligonucleotide using a compound represented by a formula (I) below or a salt thereof 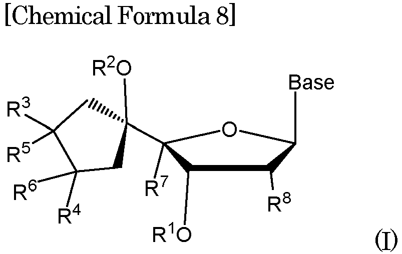 (in the formula (I),
Base represents a purin-9-yl group that may have any one or more substituents selected from an a group, or a 2-oxo-1,2-dihydropyrimidin-1-yl group that may have any one or more substituents selected from the a group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R¹ and R² each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from the a group and may include a heteroatom, an aralkyl group with an aryl moiety having 3 to 12 carbon atoms that may have any one or more substituents selected from the a group and may include a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R⁹)R¹⁰ [where R⁹ and R¹⁰ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms],
R³ and R⁴ are each independently a hydrogen atom, a hydroxy group, a hydroxy group protected by a protecting group for nucleic acid synthesis, an amino group, an amino group protected by a protecting group for nucleic acid synthesis, or an alkyl group having 1 or 2 carbon atoms,
R⁵ and R⁶ are hydrogen atoms, or R⁵ and R⁶ form a single bond together, and
R⁷ is a hydrogen atom and R⁸ is a hydrogen atom, a halogen atom; a linear alkoxy group having 1 to 6 carbon atoms that may have undergone substitution by a linear alkoxy group having 1 to 6 carbon atoms; or -OR¹¹ [where R¹¹ is a hydrogen atom or a hydroxy group protecting group for nucleic acid synthesis], or
R⁷ and R⁸ represent a divalent group -CH₂O- or -OCH₂- together).
